(19) Europäisches Patentamt / European Patent Office / Office européen des brevets

(11) **EP 3 685 850 A1**

(12) **EUROPEAN PATENT APPLICATION**
published in accordance with Art. 153(4) EPC

(43) Date of publication:
**29.07.2020 Bulletin 2020/31**

(21) Application number: **18859527.6**

(22) Date of filing: **20.09.2018**

(51) Int Cl.:
**A61K 38/29** (2006.01)   **A61K 9/08** (2006.01)
**A61P 5/18** (2006.01)

(86) International application number:
**PCT/JP2018/034889**

(87) International publication number:
**WO 2019/059303 (28.03.2019 Gazette 2019/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **22.09.2017   JP 2017182615**

(71) Applicant: **Asahi Kasei Pharma Corporation
Tokyo 100-0006 (JP)**

(72) Inventors:
• **MIYABE, Kohei
Tokyo
1000006 (JP)**
• **OSE, Atsushi
Tokyo
1000006 (JP)**
• **SATO, Yuki
Tokyo
1000006 (JP)**
• **KODAMA, Toshiyuki
Tokyo
1000006 (JP)**
• **MATSUNAWA, Yasuhiro
Tokyo
1000006 (JP)**
• **YAMAMOTO, Hikaru
Tokyo
1000006 (JP)**

(74) Representative: **Forstmeyer, Dietmar et al
BOETERS & LIECK
Oberanger 32
80331 München (DE)**

(54) **TERIPARATIDE-CONTAINING LIQUID PHARMACEUTICAL COMPOSITION HAVING EXCELLENT PHARMACODYNAMICS AND/OR STABILITY**

(57)   A liquid pharmaceutical preparation for subcutaneous administration in human containing 28.2 μg of teriparatide or a salt thereof (Component 1) in a unit dose in terms of teriparatide, wherein the Component 1 concentration is from 80 to 240 μg/mL. This liquid pharmaceutical preparation is excellent in the viewpoint of pharmacokinetics.

[FIG. 3]

FIG. 3

**Description**

TECHNICAL FIELD

The present invention relates to a liquid pharmaceutical preparation for subcutaneous administration containing teriparatide or a salt thereof.

BACKGROUND ART

PTH (parathyroid hormone) is a hormone involved in the regulation of the blood calcium concentration as with calcitonins and vitamin D. As to PTH peptides which are physiologically active equivalents of naturally occurring PTH, PTH peptide-containing freeze-dried preparations and PTH peptide-containing liquid agents have also been known.

PRIOR ART REFERENCES

PATENT PUBLICATIONS

Patent Publication 1: Japanese Patent Laid-Open No. Hei-5-306235
Patent Publication 2: Japanese Patent Laid-Open No. 2004-10511
Patent Publication 3: Japanese Patent Laid-Open No. 2007-186466
Patent Publication 4: Japanese Unexamined Patent Publication No. 2001-525372
Patent Publication 5: WO 2006/22301
Patent Publication 6: WO 2012/169435
Patent Publication 7: Japanese Unexamined Patent Publication No. 2015-504087
Patent Publication 8: Japanese Patent Laid-Open No. Sho-63-57527
Patent Publication 9: Japanese Patent Laid-Open No. Hei-2-96533
Patent Publication 10: Japanese Unexamined Patent Publication No. 2004-513069
Patent Publication 11: Japanese Patent Laid-Open No. 2005-213158
Patent Publication 12: WO 2011/139838
Patent Publication 13: Japanese Unexamined Patent Publication No. 2014-507484

NON-PATENT PUBLICATIONS

Non-Patent Publication 1: Package Insert of Teribone(Registered Trademark) Subcutaneous Injection 56.5 $\mu$g (revised November, 2015 (sixth edition, revised on the cautions and the like upon use))
Non-Patent Publication 2: Package Insert of Forteo(Registered Trademark) Subcutaneous Injection Kit 600 $\mu$g (revised July, 2014 (seventh edition))
Non-Patent Publication 3: Sung et al., Journal of Biological Chemistry, (1991), 266(5), 2831-2835
Non-Patent Publication 4: Takei et al., Peptide Chemistry 1979, (1980), 187-192
Non-Patent Publication 5: Merrifield, Advances In Enzymology, (1969), 32, 221-296
Non-Patent Publication 6: K. Ikawa et al., Jpn J Biomet, (2015), 36, Special Issue, S3-S18
Non-Patent Publication 7: Mach et al., Therapeutic Delivery, (2011), 2(6), 727-736
Non-Patent Publication 8: Kinnunen et al., Journal of Controlled Release, (2014), 182, 22-32
Non-Patent Publication 9: "Key Issues and Perspectives for Drug Metabolism and Pharmacokinetics in Drug Discovery and Development, " Sumitomo Chemical II (26 to 34)
Non-Patent Publication 10: Chen et al., Biochem. Biophys. Res. Commun., (1971), 44(6), 1285-1291
Non-Patent Publication 11: Greenfield, Nature Protocols, (2006), 1(6), 2876-2890
Non-Patent Publication 12: Lee et al., Biopolymers, (1989), 28, 1115-1127
Non-Patent Publication 13: Strickland et al., Biochemistry, (1993), 32, 6050-6057
Non-Patent Publication 14: Proceedings of Annual Meeting of the Pharmaceutical Society of Japan, 118th Annual Meeting, 1998, 4, 34
Non-Patent Publication 15: Izutsu et al., Journal of Pharmaceutical Sciences, (2006), 95(4), 781-789
Non-Patent Publication 16: H. Hiramatsu (Graduate School of Pharmaceutical Sciences and Faculty of Pharmaceutical Sciences, Tohoku University), "Secondary Structure Analysis of Proteins Using Infrared Absorption Spectroscopy," The Society of Protein Science Archive, 2009, 2, e054

Non-Patent Publication 17: K. Izutsu et al., "Tanpakushitu Iyakuhin no Hi-hakai-hyoka ni Muketa Suiyoeki to Toketsukanso-kotai-chu no Nijikozo Kento (Secondary Structure Studies on Protein Pharmaceutics in Aqueous Solutions and Freeze-Dried Solids Towards Nondestruction Evaluation)," Proceedings of 21st Near Infrared Forum Lectures, 2005, 59

Non-Patent Publication 18: Armstrong et al., Proc. Natl. Acad. Sci. USA, (1993), 90, 11337-11340

Non-Patent Publication 19: Chakrabartty et al., Biochemistry, (1993), 32(21), 5560-5565

Non-Patent Publication 20: Wu et al., Proc. Natl. Acad. Sci. USA, (1979), 76(8), 3656-3659

Non-Patent Publication 21: Aloj et al., Archives of Biochemistry and Biophysics, (1972), 150(2), 782-785

Non-Patent Publication 22: Salgin et al., International Journal of Electrochemical Science, (2012), 7, 12404-12414

Non-Patent Publication 23: Yamamoto et al., Eur J Pharmacol. (2015), 764, 457-462

Non-Patent Publication 24: Outline Document Materials for Teribone(Registered Trademark) Subcutaneous Injection 56.5 μg (http://www.pmda.go.jp/drugs/2011/P201100155/index.html)

Non-Patent Publication 25: Mitsuhiro Miyazawa, "Tokushu ni Atatte: Tanpakushitu no Rittaikozo Kaisekiho (Special Issue: Steric Structure Analysis Method of Proteins," SANSHI-KONCHU BIOTEC, 2012, 81(2), 105-106

Non-Patent Publication 26: Edited by the Pharmaceutical Society of Japan, Standard Pharmacy Series 7: Science of Producing Preparations, First Edition, First Printing, February 10, 2006, 12-13

Non-Patent Publication 27: N. Kosakaya et al., "Heikei-ki Nihonjin Josei niokeru Youtsui Kotsumitsudo no Gonenkan no Gensho ni Taisuru Kanren Inshi (Associating Factors for Loss in Lumbar Vertebrate Bone Density over a 5-Year Period in Menopausal Japanese Women), " Journal of Japan Society of Nutrition and Food Science 1999, 52(5), 307-313

Non-Patent Publication 28: H. Mizuno et al., "Maku Tokasei Pepuchido no Amino-san Hairetsu Kaihen niyoru pH Outousei no Hyoka (Evaluation of pH-Responsivity of Membrane-Permeable Peptides by Alterations of Amino Acid Sequences," Nihon University, College of Industrial Technology, Outlines of 48th Academic Meeting Lectures (2015-12-5), 543-544

Non-Patent Publication 29: Tim J et al., Protein Science, (2007), 16, 1193-1203

Non-Patent Publication 30: Leonid K., Drug Metab. Dispos., (2014), 42, 1890-1905

## SUMMARY OF THE INVENTION

### PROBLEMS TO BE SOLVED BY THE INVENTION

An object of the present invention is to provide a liquid pharmaceutical preparation for subcutaneous administration containing teriparatide or a salt thereof having excellent pharmacokinetics (for example, high bioavailability) and/or high safety (for example, suppressed development frequencies of side effects of digestive tracts).

### MEANS TO SOLVE THE PROBLEMS

In one embodiment of a liquid pharmaceutical preparation for subcutaneous administration of the present invention, the $\alpha$-helix content ratio in teriparatide or a salt thereof is within a specified range (for example, 13.0% or more).

In one embodiment of a liquid pharmaceutical preparation for subcutaneous administration of the present invention, the number of amino acid residues that form an $\alpha$-helical structure in teriparatide or a salt thereof is within a specified range (for example, 4.5 or more).

In one embodiment of a liquid pharmaceutical preparation for subcutaneous administration of the present invention, the average residue molar ellipticity $[\theta]_{222}$ as determined by circular dichroism (CD) spectroscopy (measurement wavelength: 222 nm) shown by the preparation is within a specified range (for example, -6300(deg.cm$^2$/d mol) or less).

In these liquid pharmaceutical preparations for subcutaneous administrations, excellent pharmacokinetics (for example, high bioavailability) are obtained.

In addition, in one embodiment of a liquid pharmaceutical preparation for subcutaneous administration of the present invention, a unit dose per one administration (a unit dose) of teriparatide or a salt thereof is a specified amount (for example, 28.2 μg).

Alternatively, in one embodiment of a liquid pharmaceutical preparation for subcutaneous administration of the present invention, the time to the maximum plasma concentration ($T_{max}$) of teriparatide or a salt thereof obtained by administration of a unit dose is within a specified range (for example, less than 0.7 hr).

Alternatively, in one embodiment of a liquid pharmaceutical preparation for subcutaneous administration of the present invention, the time course in a state of the plasma concentration of teriparatide or a salt thereof having a specified threshold value (for example, 250 pg/mL) or more after administration of a unit dose is within a specified range (for example, less than 1.0 hr).

In these liquid pharmaceutical preparations for subcutaneous administration, excellent safety (for example, suppressed development frequencies of side effects of digestive tracts) is obtained.

Specifically, the present invention relates to the following inventions and the like.

[1] A liquid pharmaceutical preparation for subcutaneous administration in human containing 28.2 $\mu$g of Component 1 in a unit dose in terms of teriparatide,
the Component 1 being teriparatide or a salt thereof,
wherein the Component 1 concentration is from 80 to 240 $\mu$g/mL.

[2] The liquid pharmaceutical preparation for subcutaneous administration in human according to the above [1], wherein the Component 1 concentration is from 100 to 200 $\mu$g/mL.

[3] The liquid pharmaceutical preparation for subcutaneous administration in human according to the above [1] or [2], wherein $T_{max}$ calculated by an analysis independent of pharmacokinetic models (NCA (Non Compartmental Analysis)) to the time of administration of a unit dose is from 0.5 to 0.7 (1/hr).

[4] The liquid pharmaceutical preparation for subcutaneous administration in human according to any of the above [1] to [3], wherein the time course in a state of a plasma concentration of the Component 1 of 100 pg/ml or more after administration of a unit dose is less than 2.1 (hr), and the time course in a state of a plasma concentration of the Component 1 of 250 pg/ml or more after administration of a unit dose is less than 1.0 (hr).

[5] The liquid pharmaceutical preparation for subcutaneous administration in human according to any of the above [1] to [4], for use in administration to postmenopausal women.

[6] The liquid pharmaceutical preparation for subcutaneous administration in human according to any of the above [1] to [5], wherein in the Component 1, the number of amino acid residues that form an $\alpha$-helical structure is 4.5 or more and 5.5 or less.

[7] The liquid pharmaceutical preparation according to the above [6], wherein the number of amino acid residues is the number of amino acid residues on the basis of the $\alpha$-helix content ratio estimated using the following Estimation formula 1 from the numerical value a of the average residue molar ellipticity obtained by circular dichroism (CD) spectroscopy satisfying the following Measurement conditions 1 to 4:

Measurement condition 1: a measurement wavelength of 222 nm;
Measurement condition 2: a sample concentration (Component 1 concentration) of from 0.1 to 0.3 mg/mL;
Measurement condition 3: a measurement temperature of 20°C; and Measurement condition 4: a cell length of from 1 to 2 mm;

$$\text{Estimation formula 1:}$$
$$\alpha\text{-Helix Content Ratio} = \frac{-(\text{Numerical Value } \mathbf{a} + 2340)}{30300}.$$

[8] The liquid pharmaceutical preparation for subcutaneous administration in human according to any of the above [1] to [7], wherein the Component 1 is teriparatide acetate.

[9] The liquid pharmaceutical preparation for subcutaneous administration in human according to any of the above [1] to [8], wherein the liquid pharmaceutical preparation for subcutaneous administration in human is an aqueous pharmaceutical preparation for subcutaneous administration in human (excluding reconstructs of freeze-dried preparations).

[10] The liquid pharmaceutical preparation for subcutaneous administration in human according to any of the above [1] to [9], wherein the liquid pharmaceutical preparation for subcutaneous administration in human is an aqueous pharmaceutical preparation for subcutaneous administration in human, and its solvent is a water for injection.

ADVANTAGEOUS EFFECTS OF THE INVENTION

According to the present invention, a liquid pharmaceutical preparation containing teriparatide or a salt thereof having excellent pharmacokinetics and/or safety is provided.

BRIEF DESCRIPTION OF THE DRAWINGS

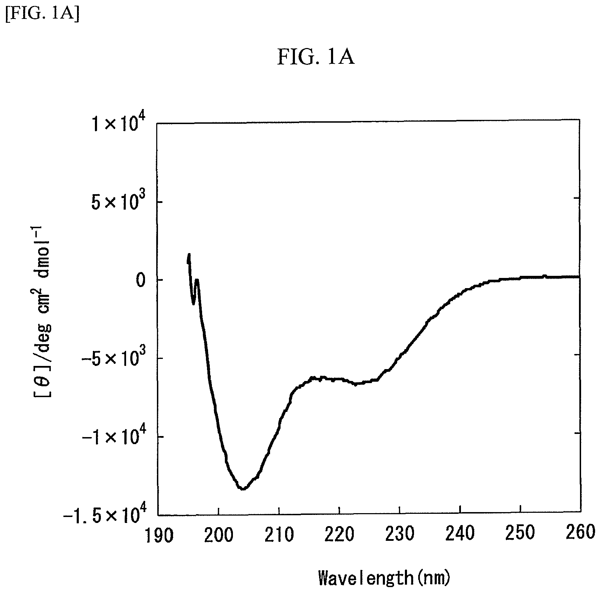

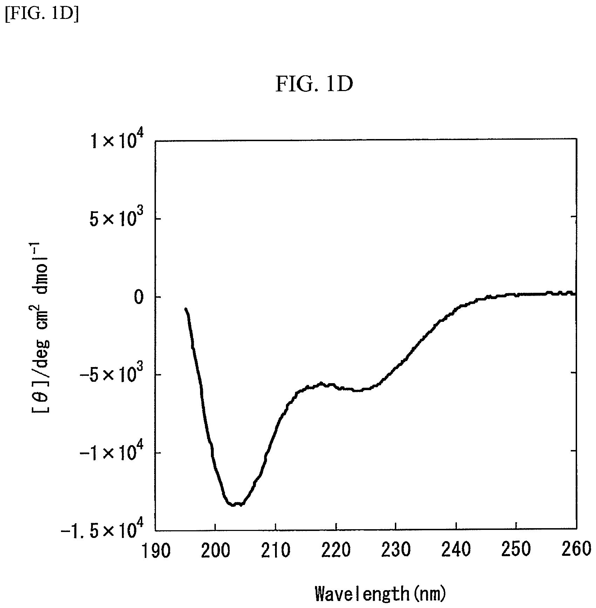

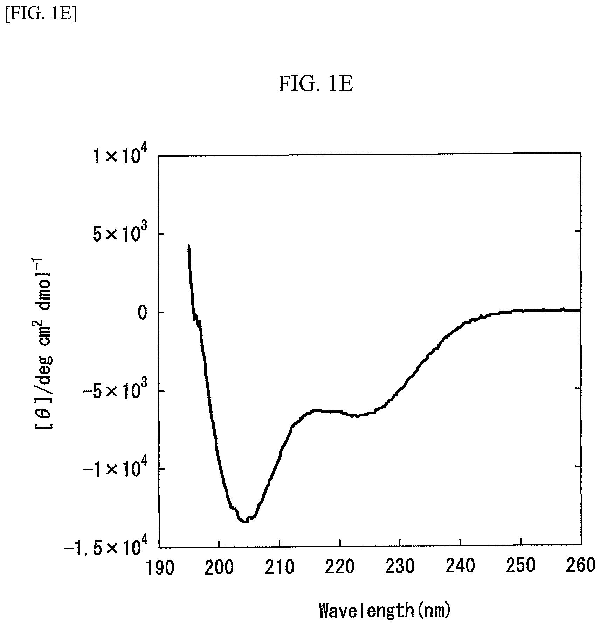

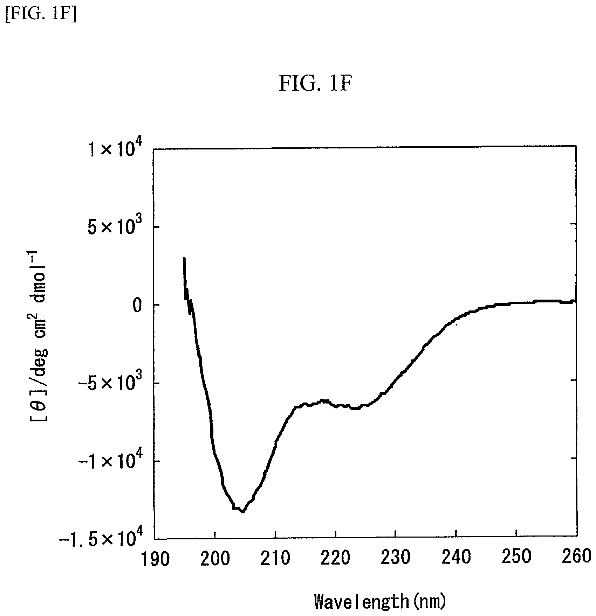

[FIG. 1A] FIG. 1A is a graph showing the measurement results obtained by carrying out circular dichroism (CD) spectroscopy by 8 accumulations at 20°C, using Formulation A prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

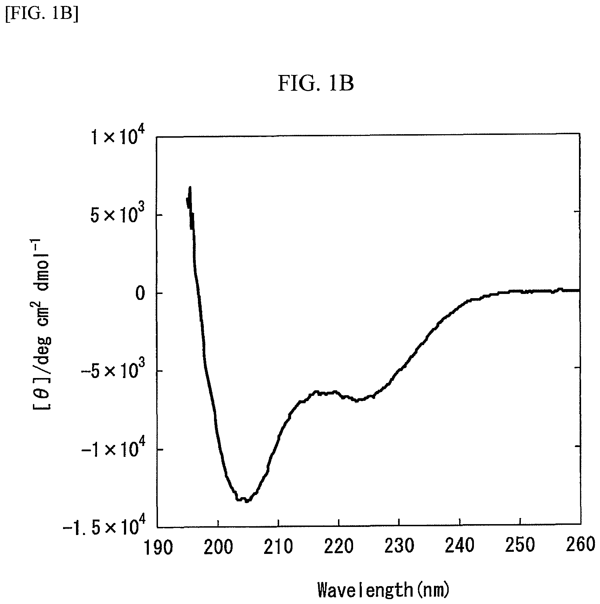

[FIG. 1B] FIG. 1B is a graph showing the measurement results obtained by carrying out the circular dichroism (CD) spectroscopy by 8 accumulations at 20°C, using Formulation B prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

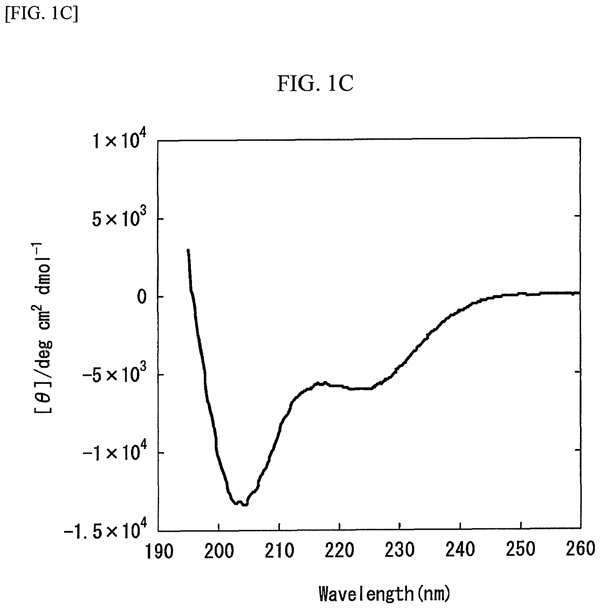

[FIG. 1C] FIG. 1C is a graph showing the measurement results obtained by carrying out the circular dichroism (CD) spectroscopy by 8 accumulations at 20°C, using Formulation C prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

[FIG. ID] FIG. ID is a graph showing the measurement results obtained by carrying out the circular dichroism (CD) spectroscopy by 8 accumulations at 20°C, using Formulation D prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

[FIG. IE] FIG. IE is a graph showing the measurement results obtained by carrying out the circular dichroism (CD) spectroscopy by 8 accumulations at 20°C, using Formulation E prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

[FIG. IF] FIG. IF is a graph showing the measurement results obtained by carrying out the circular dichroism (CD) spectroscopy by 8 accumulations at 20°C, using Formulation F prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

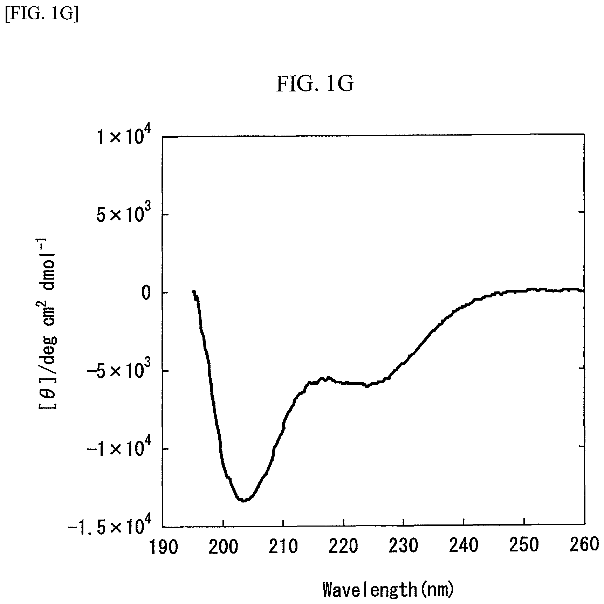

[FIG. 1G] FIG. 1G is a graph showing the measurement results obtained by carrying out the circular dichroism (CD) spectroscopy by 8 accumulations at 20°C, using Formulation G prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

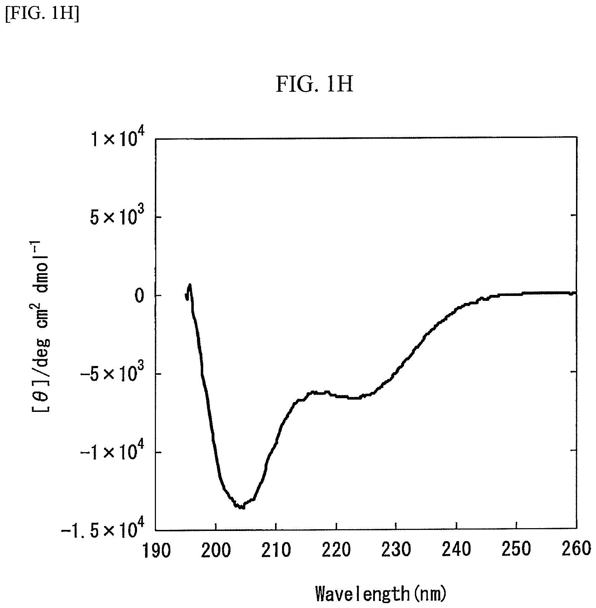

[FIG. 1H] FIG. 1H is a graph showing the measurement results obtained by carrying out the circular dichroism (CD) spectroscopy by 8 accumulations at 20°C, using Formulation H prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm), and the axis of ordinates "[$\theta$]/deg.cm$^2$ d mol$^{-1}$" is an average residue molar ellipticity [$\theta$].

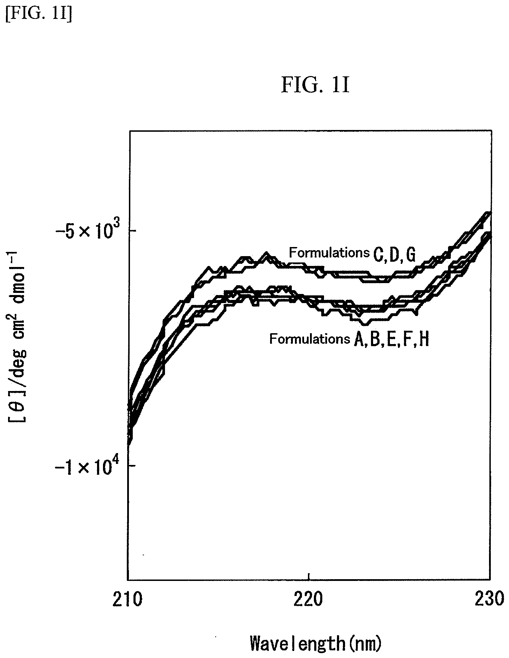

[FIG. 1I] FIG. 1I is a graph showing the measurement results obtained by carrying out the circular dichroism (CD) spectroscopy by 8 accumulations at 20°C, using Formulation I prepared in "Preparation of Liquid Pharmaceutical Preparations Subjected to Test for Circular Dichroism (CD) Spectroscopy" as a measurement subject. The axis of abscissas "Wavelength (nm)" is a measurement wavelength (nm) (210 to 230 nm), and the axis of ordinates "[$\theta$]/deg.cm$^2$ d mol$^{-1}$" is an average residue molar ellipticity [$\theta$].

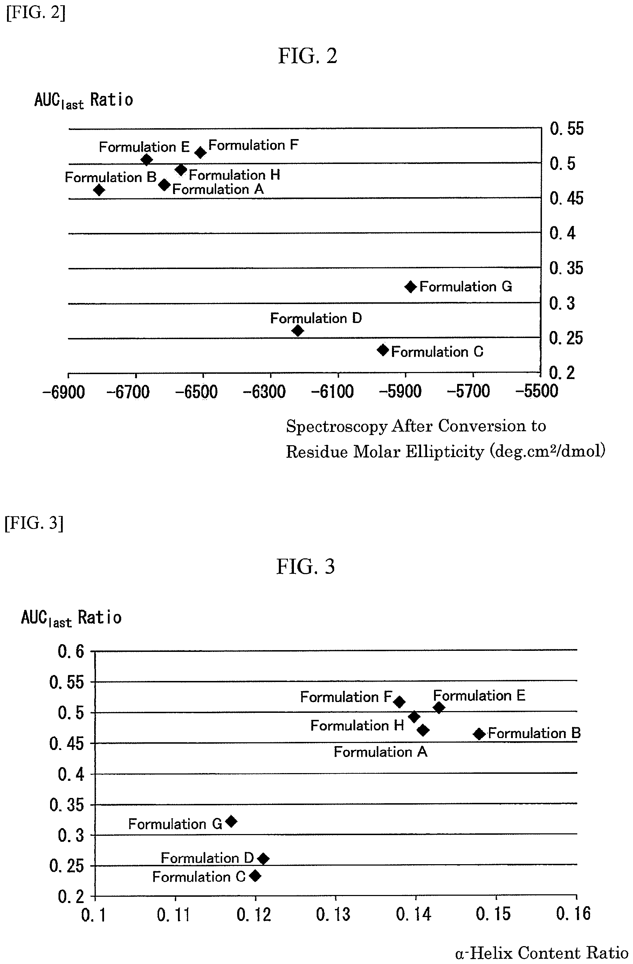

[FIG. 2] FIG. 2 is a graph collectively showing the results obtained by carrying out the test for circular dichroism (CD) spectroscopy and the pharmacokinetic tests in human (Example 3: Pharmacokinetic Test in Human (2)) using Formulations A to H (a total of 8 formulations) prepared in "Preparation of Liquid Pharmaceutical Preparations Subjected to Test for Circular Dichroism (CD) Spectroscopy" as subjects. The results of the tests for the circular dichroism (CD) spectroscopy are shown as the measurement results of the measurement 2 of the same test (average residual molar ellipticity [$\theta$]$_{222}$), and the pharmacokinetic test results in human are shown as AUC$_{last}$ Ratio, which is defined as a ratio of each formulation based on Control Formulation 2 with respect to AUC$_{last}$ (area under the plasma concentration versus(-) time curve until the last observation time).

[FIG. 3] FIG. 3 is a graph collectively showing the results obtained by carrying out the tests for circular dichroism (CD) spectroscopy and the pharmacokinetic tests in human (Example 3: Pharmacokinetic Test in Human (2)) using Formulations A to H (a total of 8 formulations) prepared in "Preparation of Liquid Pharmaceutical Preparations Subjected to Test for Circular Dichroism (CD) Spectroscopy" as subjects. The results of the tests for the circular dichroism (CD) spectroscopy are shown as the measurement results of the measurement 2 of the same test ($\alpha$-

helix content ratio), and the pharmacokinetic test results in human are shown as $AUC_{last}$ Ratio, which is defined as a ratio of each formulation based on Control Formulation 2 with respect to $AUC_{last}$ (area under the plasma concentration versus(-) time curve until the last observation time).

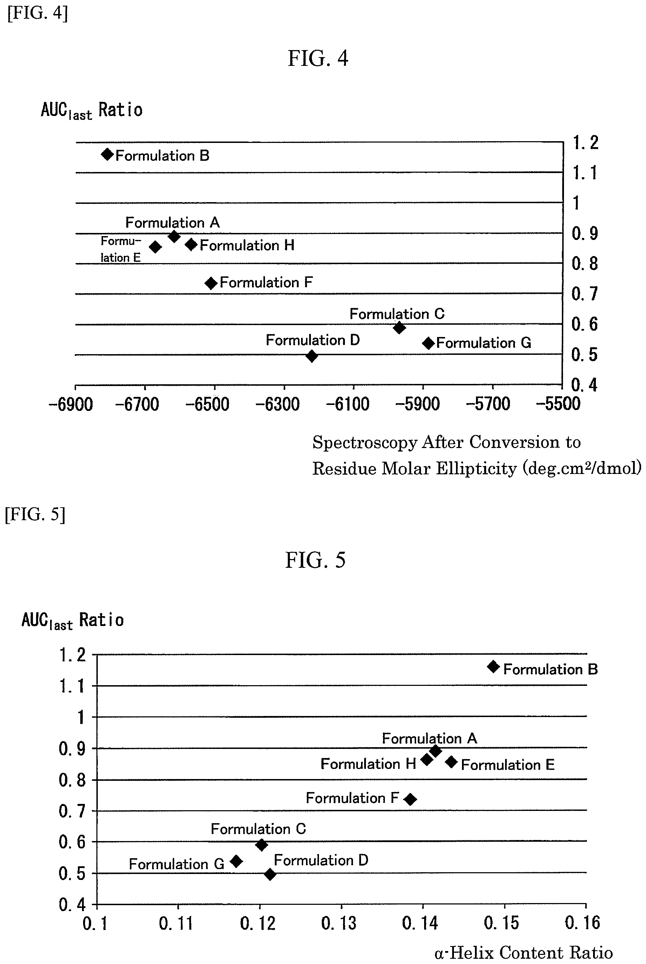

[FIG. 4] FIG. 4 is a graph collectively showing the results obtained by carrying out the tests for circular dichroism (CD) spectroscopy and the pharmacokinetic tests in monkeys (Example 2: Pharmacokinetic Test in Monkeys) using Formulations A to H (a total of 8 formulations) as subjects. The results of the tests for the circular dichroism (CD) spectroscopy are shown as the measurement results of the measurement 2 of the same test (average residual molar ellipticity $[\theta]_{222}$), and the pharmacokinetic test results in monkeys are shown as $AUC_{last}$ Ratio, which is defined as a ratio of each formulation with respect to $AUC_{last}$ (area under the plasma concentration versus(-) time curve until the last observation time) based on Control Formulation 1.

[FIG. 5] FIG. 5 is a graph collectively showing the results obtained by carrying out the tests for circular dichroism (CD) spectroscopy and the pharmacokinetic tests in monkeys (Example 2: Pharmacokinetic Test in Monkeys) using Formulations A to H (a total of 8 formulations) as subjects. The results of the tests for the circular dichroism (CD) spectroscopy are shown as the measurement results of the measurement 2 of the same test ($\alpha$-helix content ratio), and the pharmacokinetic test results in monkeys are shown as $AUC_{last}$ Ratio, which is defined as a ratio of each formulation based on Control Formulation 1 with respect to $AUC_{last}$ (area under the plasma concentration versus(-) time curve until the last observation time).

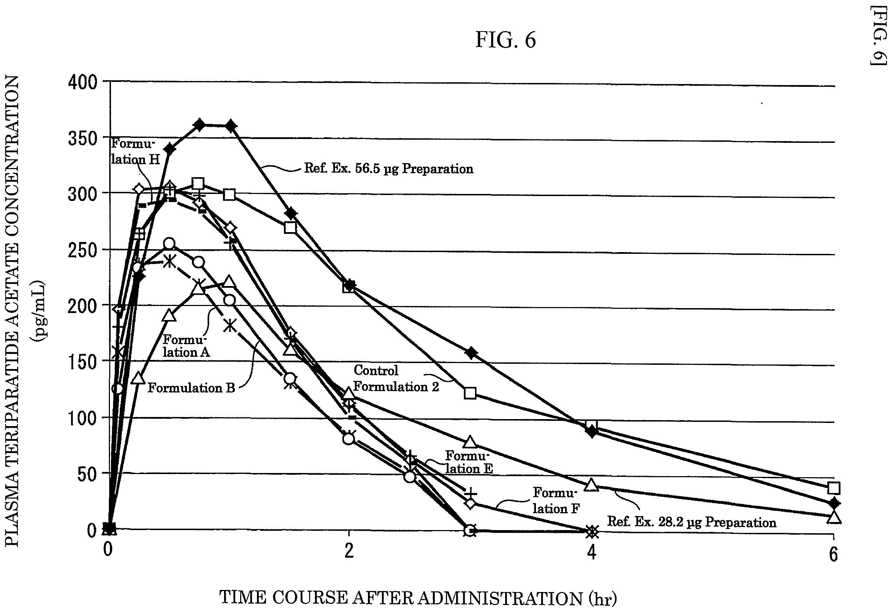

[FIG. 6] FIG. 6 is a graph showing the time transition of plasma teriparatide acetate concentrations obtained by administering each of Formulations A, B, E, F, and H subjected to Examples, and 28.2 $\mu$g preparation and 56.5 $\mu$g preparation subjected to Reference Example (Reference Example concerning the invention in which $T_{max}$ of Component 1 is within a specified range).

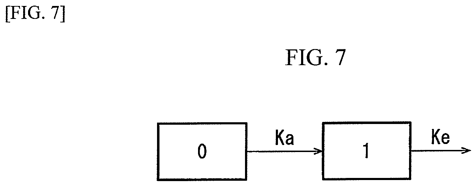

[FIG. 7] FIG. 7 is a schematic view of a pharmacokinetic model (one-compartment model) used in Examples 6 and 7, wherein Ka is an absorption rate constant, and Ke is an elimination rate constant.

## MODES FOR CARRYING OUT THE INVENTION

The present invention shall be described hereinafter in detail on the basis of specific embodiments. However, the present invention is not intended to be bound to the following embodiments, and can be carried out in any embodiments within the range that would not depart from the spirit of the present invention.

### 1. Liquid Pharmaceutical Preparation for Subcutaneous Administration:

The present invention provides, as one embodiment, a liquid pharmaceutical preparation for subcutaneous administration containing teriparatide or a salt thereof as Component 1, wherein the $\alpha$-helix content ratio of the Component 1 in the above preparation is within a specified range.

The present invention provides, as one embodiment, a liquid pharmaceutical preparation for subcutaneous administration containing teriparatide or a salt thereof as Component 1, wherein the number of amino acid residues that form an $\alpha$-helical structure in the Component 1 in the above preparation is within a specified range.

The present invention provides, as one embodiment, a liquid pharmaceutical preparation for subcutaneous administration containing teriparatide or a salt thereof as Component 1, wherein the average residue molar ellipticity $[\theta]_{222}$ as determined by circular dichroism (CD) spectroscopy (measurement wavelength: 222 nm) shown by the preparation is -6300 (deg.cm$^2$/d mol) or less.

In addition, the present invention provides, as another embodiment, a liquid pharmaceutical preparation for subcutaneous administration containing teriparatide or a salt thereof as Component 1, wherein the unit dose of the teriparatide or a salt thereof is a specified amount.

Further, the present invention provides, as another embodiment, a liquid pharmaceutical preparation for subcutaneous administration containing teriparatide or a salt thereof as Component 1, wherein the $T_{max}$ of Component 1 obtained in administration of a unit dose is within a specified range.

Alternatively, the present invention provides, as another embodiment, a liquid pharmaceutical preparation for subcutaneous administration containing teriparatide or a salt thereof as Component 1, wherein the time course in a state of the plasma concentration of teriparatide or a salt thereof having a specified threshold value or higher after administration of a unit dose is within a specified range.

### (1) Liquid Pharmaceutical Preparation:

A liquid pharmaceutical preparation of the present invention is not particularly limited in its form, so long as the liquid pharmaceutical preparation is a liquid pharmaceutical preparation for subcutaneous administration containing teriparatide or a salt thereof (Component 1) described later. Example of the liquid pharmaceutical preparation of the present invention include subcutaneous injections and subcutaneous insert capsules. The liquid pharmaceutical preparation of the present invention is not particularly limited in its container, needles, wrappings, or the like, so long as the liquid pharmaceutical preparation is used for subcutaneous administration. The term "pharmaceutical preparation" as used herein means a drug used in prevention/treatment/diagnosis of a given disease to a mammal (human, monkey, rat, or the like). As the pharmaceutical preparation, examples of the pharmaceutical preparation for human are preferred. In a case where the subject to be administered is human, sex, age, and the presence or kinds of suffering diseases thereof are not particularly limited, and, for example, the subjects can be postmenopausal women.

The solvent used in a liquid pharmaceutical preparation of the present invention may be, but not particularly limited to, an aqueous solvent or a non-aqueous solvent, and it is preferred to contain an aqueous solvent, and the solvent may be substantially constituted only by an aqueous solvent. It is preferable that the present invention is an aqueous pharmaceutical preparation. A liquid pharmaceutical preparation or a solvent (aqueous solvent or the like) may contain various components such as inorganic salts, organic salts, buffer, and additives, within the range that would not depart from the spirit of the present invention. For example, the liquid pharmaceutical preparation can be prepared with a water for injection, physiological saline, or the like.

As a liquid pharmaceutical preparation of the present invention, examples include preferably an aqueous pharmaceutical preparation for subcutaneous administration in human, and most preferably an aqueous pharmaceutical preparation for subcutaneous injection in human. Here, when the liquid pharmaceutical preparation of the present invention is a preparation for subcutaneous administration, the site of subcutaneous administration is preferably, but not particularly limited to, sites that have smaller distributions of nerves or blood vessels, larger subcutaneous fats, and no bones. Such sites preferably include abdominal parts, upper arm parts, femur parts, and hip parts, and abdominal parts are preferred.

(2) Teriparatide or Salt Thereof (Component 1):

In the present invention, human PTH(1-34) is a peptide represented by a partial amino acid sequence consisting of amino acid residues of the position 1 to the position 34 from the N-terminal side in the amino acid sequence of human PTH(1-84) which is human parathyroid hormone.

In the present invention, teriparatide means human PTH(1-34) in a free form. Teriparatide can be in a salt form.

In the present invention, the salt of teriparatide includes any salts formed by teriparatide and one or more volatile organic acids. Examples of the volatile organic acid include trifluoroacetic acid, formic acid, acetic acid, and the like. When teriparatide in a free form and the volatile organic acid form a salt, the ratio thereof is not particularly limited so long as the salt is formed. In particular, as the volatile organic acid, acetic acid is preferred. Specifically, as the salt of teriparatide in the present invention, teriparatide acetate is preferably exemplified.

Since teriparatide or a salt thereof is a peptide, it has an isoelectric point (pI). The measurement of pI can be carried out by a method that itself is known (for example, a method using HPLC or electrophoresis or the like). In general, the pI of teriparatide or a salt thereof is known to be from 8.3 to 8.4.

Teriparatide or a salt thereof (Component 1) can be produced by methods that themselves are known (for example, methods described in Non-Patent Publications 3 to 5 and the like).

(3) Content, Usage, and Concentration of Teriparatide or Salt Thereof (Component 1):

The amount of teriparatide or a salt thereof (Component 1) contained in the liquid pharmaceutical preparation of the present invention is not particularly limited, and examples of the amount include preferably as follows. Specifically, the amount of the Component 1 in the preparation is preferably 10 μg or more, more preferably 20 μg or more, 25 μg or more, 27 μg or more, and even more 28 μg or more. In addition, the amount of the Component 1 in the preparation is preferably 100 μg or less, more preferably 50 μg or less, 40 μg or less, 35 μg or less, and even more 30 μg or less. In particular, the content of the Component 1 is preferably 28.2 μg or 29.2 μg, in terms of teriparatide. When teriparatide used is an acetate, examples include the amount added with the acetate amount. For example, in a case where teriparatide pentaacetate is used, the content of the Component 1 is preferably 30.3 μg or 31.3 μg, in terms of teriparatide pentaacetate.

The unit dose of teriparatide or a salt thereof (Component 1) contained in the liquid pharmaceutical preparation of the present invention is not particularly limited, and examples of the unit dose include preferably as follows. Specifically, the unit dose of the Component 1 of the preparation is more preferably 25 μg or more, 27 μg or more, and even more 28 μg or more. In addition, the unit dose of the Component 1 of the preparation is more preferably 35 μg or less, 30 μg or less, and even more 29 μg or less. In particular, the unit dose of the Component 1 is preferably 28.2 μg, in terms of teriparatide. In particular, excellent safety accompanying administration of a unit dose is preferably obtained by having a unit dose of the Component 1 of the above upper limit or lower. In addition, examples include an embodiment of having a unit dose of Component 1 of 56.5 μg.

Examples of the concentration of teriparatide or a salt thereof (Component 1) contained in the liquid pharmaceutical preparation of the present invention include, but not particularly limited to, preferably as follows. Specifically, the concentration of the Component 1 in the preparation is preferably 50 μg/mL or more, and more preferably 70 μg/mL or more, 80 μg/mL or more, 100 μg/mL or more, exceeding 100 μg/mL, 110 μg/mL or more, and even more 120 μg/mL or more. In addition, the concentration of the Component 1 in the preparation is preferably 500 μg/mL or less, and more preferably 250 μg/mL or less, less than 250 μg/mL, 240 μg/mL or less, 200 μg/mL or less, 180 μg/mL or less, and even more 160 μg/mL or less. In particular, an example of 141 μg/mL is most preferred. A high absorption rate of the Component 1 and excellent safety accompanying administration of a unit dose of this preparation are obtained by adjusting the concentration of the Component 1 to the above range. Here, in a case where the Component 1 is a teriparatide salt, it is preferable that the concentration of the Component 1 is in terms of the concentration of its free from (teriparatide).

(4) α-Helix Content Ratio and Number of Amino Acid Residues That Form α-Helix in Teriparatide or Salt Thereof (Component 1):

In the present invention, the α-helix content ratio of the Component 1 (teriparatide or a salt thereof) means a proportion of an average number of amino acid residues (a number corresponding to amino acid residues) that form the α-helical structure to the entire number of amino acid residues (entire number of residues: specifically 34) owned by the Component 1 contained in the liquid pharmaceutical preparation of the present invention. The proportion may be shown as a value calculated by dividing the number of corresponding residues by the entire number of residues (0 to 1), or may be calculated in terms of percentage (0 to 100(%)). For example, the α-helix content ratio of the Component 1 of 13% means that about 4.42 (= 0.13 × 34) of the amino acid residues in an average out of 34 amino acid residues of the Component 1 form an α-helical structure.

Here, in the Component 1 contained in the liquid pharmaceutical preparation of the present invention, many molecular species are present with regard to the formation sites of the α-helical structures and the amounts thereof, which may be dynamically equilibrated therebetween, and many Component 1 contained in the liquid pharmaceutical preparation of the present invention may show substantially the same formation site of the α-helical structure and the amount thereof. In any case, the α-helix content ratio means a proportion of the number of amino acid residues that form an α-helical structure of the Component 1 to the entirety of the number of amino acid residues owned by the Component 1.

In the present invention, it is possible to estimate the α-helix content ratio of the Component 1 contained in the liquid pharmaceutical preparation in accordance with, for example, circular dichroism (CD) spectroscopy (see, Non-Patent Publications 10 and 11 or the like). For example, it is preferable that a circular dichroism (CD) spectroscopy value ([m deg]) is obtained at a measurement wavelength of 222 nm using a liquid pharmaceutical preparation containing the Component 1 as a sample, and its measurement value is converted to an average residue molar ellipticity ([deg.cm$^2$/d mol]) to estimate an α-helix content ratio of the Component 1 from the following mathematical formula using a numerical value a of the average residual molar ellipticity obtained.

[Math Formula 1]

$$\alpha\text{-Helix Content Ratio} = \frac{-(\text{Numerical Value } \mathbf{a} + 2340)}{30300}$$

(Non-Patent Publication 10)

The measurement conditions are not particularly limited, and, for example, the content ratio can be measured under the following conditions.

1) a measurement wavelength of 222 nm;
2) a sample concentration (Component 1 concentration) of from 0.1 to 0.3 mg/mL;
3) a temperature of 20°C; and
4) a cell length of from 1 to 2 mm.

A sample volume can be appropriately selected, which may be, for example, 0.5 mL or so. The apparatus for the CD spectroscopy is not particularly limited, and, for example, a circular dichroism spectrometer (J-720; sold by JASCO CORPORATION) can be used.

In addition, in a case where a liquid pharmaceutical preparation contains a high-concentration amino acid or the like as an additive, the background level becomes high, whereby consequently may make it difficult to measure the α-helix content ratio in accordance with the circular dichroism (CD) spectroscopy method. In such cases, the measurement may be taken by using, for example, a nuclear magnetic resonance method (NMR) in place of the CD spectroscopy method.

However, in general, when the $\alpha$-helix content ratio of the Component 1 is estimated from the circular dichroism (CD) spectroscopy results, the estimation values of the $\alpha$-helix content ratios could vary depending upon the estimation formula used in the estimation. In addition, even when the identical liquid pharmaceutical composition is used as a subject, the estimation value of the $\alpha$-helix content ratio in accordance with the NMR method may differ from the estimation value of the $\alpha$-helix content ratio in accordance with the CD method. For example, depending upon the estimation formulas used when estimating the $\alpha$-helix content ratio in accordance with the CD method, the former may be higher than the latter.

Therefore, when the NMR method is used, it is preferable to use a liquid pharmaceutical preparation of which $\alpha$-helix content ratio is estimated in accordance with the CD spectroscopy method as a control product, and to obtain a chemical shift of $C\alpha$ obtained by NMR for the same control product, and to compensate the numerical value by the divergence of the contents in accordance with both the measurement methods.

Besides the above, the $\alpha$-helix content ratio of the Component 1 contained in the liquid pharmaceutical preparation can also be measured by using methods such as ATR-FT IR (Attenuated Total Reflection of Fourier Transformer Infrared Spectroscopy), IR (infrared spectroscopy, see, Non-Patent Publication 16), Raman spectroscopy, and the like. Here, when these measurements are applied, it is necessary that a test composition to be measured is prepared so that the Component 1 is contained at a concentration of at least 1% (w/v) or more.

Even in the measurement of the $\alpha$-helix content ratio of the Component 1 in accordance with the NMR method, it is preferable that the concentration of the Component 1 in the liquid pharmaceutical preparation subjected to the test is properly adjusted to a concentration suitable for the measurement (Non-Patent Publication 25). For example, the measurement in accordance with the NMR method can be carried out by properly adjusting a concentration of the Component 1 in the liquid pharmaceutical preparation so that a concentration of the Component 1 is from 0.5 to 4 mM.

The $\alpha$-helix content ratio of the Component 1 contained in a liquid pharmaceutical preparation of the present invention is, but not particularly limited to, preferably 13% or more. In particular, the more preferred examples include 13.5% or more or 13.8% or more. A liquid pharmaceutical preparation showing excellent pharmacokinetics is obtained by having an $\alpha$-helix content ratio of the Component 1 contained in the liquid pharmaceutical preparation of the above lower limit or more.

The $\alpha$-helix content ratio of the Component 1 contained in the liquid pharmaceutical preparation of the present invention may usually satisfy the lower limit defined above (13% or more, 13.5% or more, 13.8% or more, or the like). The upper limit thereof is not particularly limited, and preferred examples include, for example, 100% or less, 80% or less, 60% or less, 50% or less, 40% or less, 30% or less, 25% or less, 20% or less, 18% or less, 16% or less, or 15.8% or less.

The number of amino acid residues that form $\alpha$-helix of the Component 1 contained in the liquid pharmaceutical preparation of the present invention can be, but not particularly limited to, selected from the range of 4 or more, and the number of the amino acid residues may be preferably 4.2 or more, 4.4 or more, 4.42 or more, and 4.5 or more. In particular, examples include more preferably 4.59 or more, 4.6 or more, 4.69 or more, and 4.7 or more. A liquid pharmaceutical preparation for subcutaneous administration showing excellent pharmacokinetics is obtained by having the number of amino acid residues that form $\alpha$-helix in Component 1 contained in the liquid pharmaceutical preparation of the lower limit defined above or more.

The number of amino acid residues that form $\alpha$-helix of Component 1 contained in the liquid pharmaceutical preparation of the present invention may usually satisfy the above lower limit (4.2 or more, 4.5 or more or the like). An upper limit thereof is not particularly limited, and may be, for example, 34 or less, 30 or less, 25 or less, 20 or less, 18 or less, 16 or less, 15 or less, 12 or less, 10 or less, 9 or less, 8 or less, 7 or less, 6.8 or less, 6.5 or less, 6.1 or less, 5.5 or less, 5.44 or less, 5.4 or less, and 5.37 or less.

The upper limit of the average residue molar ellipticity [$\theta$] in accordance with the circular dichroism (CD) spectroscopy (measurement wavelength: 222 nm) shown by the liquid pharmaceutical preparation of the present invention is not particularly limited, and examples include, for example, -6000 or less, -6100 or less, -6300 or less, and -6400 or less, and particularly preferably -6300 or less. Similarly, the lower limit thereof is not particularly limited, and examples include, for example, preferably -8000 or more, -7500 or more, -7300 or more, -7200 or more, or -7100 or more. A liquid pharmaceutical preparation for subcutaneous administration showing excellent pharmacokinetics is obtained by having an average residue molar ellipticity [$\theta$] in accordance with the circular dichroism (CD) spectroscopy (measurement wavelength: 222 nm) shown by the liquid pharmaceutical preparation of the above upper limit or less.

Here, in the present invention, the means of adjusting or increasing the $\alpha$-helix content ratio or the number of amino acid residues that form $\alpha$-helix in the Component 1 in the liquid pharmaceutical preparation is not particularly limited, and examples include the matters that a liquid pharmaceutical preparation of the present invention does not substantially contain a buffer, that an ionic compound or an ionic substance (sodium chloride or the like) is properly added, that a pH is adjusted, and the like (see also, "(2) Preparation of Liquid Pharmaceutical Preparations Subjected to Pharmacokinetic Test in Human" in Example 1, and Examples 3 and 4 given later; Non-Patent Publication 18, Non-Patent Publication 20, and the like).

Alternatively, by a means of lowering a polarity of a liquid pharmaceutical preparation of the present invention, specifically, adding various alcohols to a composition, the α-helix content ratio or the number of amino acid residues that form α-helix in Component 1 in the composition can be increased. As an alcohol having a strong ability of forming α-helix, trifluoroethanol (TFE) has been known (Non-Patent Publication 19). Isopropanol or ethanol which is used as a pharmaceutical additive is added to a liquid pharmaceutical preparation of the present invention in place of TFE, whereby the α-helix content ratio or the number of amino acid residues that form α-helix in Component 1 in the composition can be increased.

In addition, the α-helix content ratio or the number of amino acid residues that form α-helix in Component 1 in the composition can be increased by adding calcium ions ($Ca^{2+}$) to a liquid pharmaceutical preparation of the present invention (Non-Patent Publication 20). The amount of the calcium ions is not particularly limited, and it is preferable that $Ca^{2+}$ is added in an amount about 100 to about 1,000 times the concentration of the Component 1.

Here, Patent Publication 5 discloses that if a sodium acetate buffer is added to a drug solution, the bioavailability (BA) of the physiologically active peptide in the drug solution is improved as compared to that without addition (Example 2). On the other hand, in a liquid pharmaceutical preparation of the present invention, excellent pharmacokinetics are obtained without substantially containing a buffer (more specifically an acetate buffer).

(5) $T_{max}$ of Teriparatide or Salt Thereof (Component 1):

The time to the maximum plasma concentration ($T_{max}$; hr) of Component 1 obtained when a liquid pharmaceutical preparation of the present invention is subcutaneously administered in a unit dose is not particularly limited, and examples of the time to the maximum plasma concentration include preferably as follows.

Specifically, $T_{max}$ calculated in accordance with an analysis independent of pharmacokinetic models (NCA (Non Compartmental Analysis)) is preferably 0.75 (hr) or less, and more preferably 0.7 (hr) or less, 0.65 (hr) or less, 0.625 (hr) or less, 0.6 (hr) or less, or 0.5 (hr) or less. In addition, the $T_{max}$ calculated in accordance with an analysis independent of pharmacokinetic models (NCA (Non Compartmental Analysis)) is more preferably 0.1 (hr) or more, 0.2 (hr) or more, 0.25 (hr) or more, 0.3 (hr) or more, 0.4 (hr) or more, or 0.5 (hr) or more. In particular, the time to the maximum plasma concentration of from 0.5 to 0.7 (hr), and from 0.5 to 0.625 (hr) is preferred. An excellent safety accompanying administration of unit dose is preferably shown by having $T_{max}$ of the Component 1 within the above range.

Alternatively, $T_{max}$ calculated in accordance with the 1-Compartmental (Pharmacokinetics) Model Analysis is preferably 0.6 (hr) or less, more preferably 0.55 (hr) or less, or 0.5 (hr) or less. In addition, the $T_{max}$ calculated in accordance with the 1-Compartmental (Pharmacokinetics) Model Analysis is more preferably 0.1 (hr) or more, 0.2 (hr) or more, 0.25 (hr) or more, 0.3 (hr) or more, or 0.35 (hr) or more. In particular, it is preferable that the time to the maximum plasma concentration is from 0.3 to 0.6 (hr), or from 0.35 to 0.5 (hr). Excellent safety accompanying administration of a unit dose is preferably shown by having $T_{max}$ of the Component 1 within the above range.

The method for adjusting $T_{max}$ of the Component 1 obtained when a liquid pharmaceutical preparation of the present invention is subcutaneously administered in a unit dose to be within the above range is not particularly limited.

In absorption, distribution, metabolism, and elimination of a drug characterizing the pharmacokinetics of the drug (which may be called ADME from each of the capital letters of absorption, distribution, metabolism, and elimination), $T_{max}$ is generally defined by an absorption rate constant (ka) and an elimination rate constant (kel) of the drug, and calculated by the following formula using a representative model.

[Math Formula 2]

$$T_{max} = \ln (ka / kel) / (ka - kel)$$

provided that ka ≠ kel.

In Examples of the present invention, $T_{max}$ of the Component 1 obtained when a liquid pharmaceutical preparation of the present invention is subcutaneously administered in a unit dose showed a small value as compared to $T_{max}$ of the Component 1 obtained when a known Component 1 preparation is subcutaneously administered in a unit dose, and kel is considered to have a lower compositional dependency of the preparation as compared with that of ka. Taking into considerations of the above, as a method of adjusting $T_{max}$ of the Component 1 in the present invention to be within the above range, examples include preferably a method of increasing ka of the Component 1 (specifically, increasing an absorption rate of the Component 1).

Considerations can be taken on a possibility that the ionization of the Component 1 contained in the liquid pharmaceutical preparation of the present invention influences the absorption of the Component 1 when subcutaneously administered. Therefore, in order to adjust $T_{max}$ of the Component 1 to be within the above range, the Component 1 contained in the liquid pharmaceutical preparation of the present invention can be a salt with one or more volatile organic acids, or a pH of the liquid pharmaceutical preparation of the present invention can be properly adjusted in reference to Examples set forth below. Also, in order to adjust $T_{max}$ of the Component 1 to be within the above range, an additive of the liquid pharmaceutical preparation of the present invention can be properly selected in reference to Examples set forth below.

In addition, in order to adjust $T_{max}$ of the Component 1 to be within the above range, the concentration of the Component 1 contained in the liquid pharmaceutical preparation of the present invention is preferably properly regulated within the above range, and the concentration can be regulated to, for example, from 80 to 240 $\mu$g/mL, from 100 to 200 $\mu$g/mL, from 109 to 190 $\mu$g/mL, or from 120 to 160 $\mu$g/mL.

In general, it is known that the molecular weight of a drug, the additives in the drug, the analgesic, heating, pressing or the like influences the absorption rate or absorption amount of the subcutaneously administered drug (Non-Patent Publication 30).

In addition, the absorption rate constant (Ka) of Component 1 obtained when a liquid pharmaceutical preparation is subcutaneously administered to a subject to be administered becomes large by increasing the concentration of the Component 1 contained in the liquid pharmaceutical preparation (Non-Patent Publication 26). $T_{max}$ of the Component 1 can be shortened by the increase of Ka of Component 1.

Also, in the present invention, in a case where a subject to be administered is human, the human to which a liquid pharmaceutical preparation of the present invention is administered can be, for example, postmenopausal women, in order to adjust $T_{max}$ of Component 1 within the above range.

$T_{max}$ of the Component 1 obtained when a liquid pharmaceutical preparation of the present invention is subcutaneously administered in a unit dose can be confirmed in accordance with a method that self is known. The site of subcutaneous administration is preferably, but not particularly limited to, sites that have smaller distributions of nerves or blood vessels, larger subcutaneous fats, and no bones. Such sites preferably include abdominal parts, upper arm parts, femur parts, and hip parts, and abdominal parts are most preferred.

When $T_{max}$ of the Component 1 is measured, it is preferable to secure a sufficient number of measurement time points. As shown in various evaluation procedures in Examples set forth below, for example, it is preferable that blood samples are collected before the administration, and after 5, 15, 30, and 45 minutes, and after 1, 1.5, 2, 3, 4, and 6 hours of administration to measure a plasma concentration of the Component 1.

(6) Time During Which Concentration of Teriparatide or Salt Thereof (Component 1) Exceeding Specified Threshold Value Is Maintained:

The effects of a drug generally tend to be strong when the blood concentration becomes high. For example, in a case of a time-dependent antibacterial agent, the time above MIC (the transition time at a higher blood concentration than the minimum inhibitory concentration (MIC)) is important in its action.

On the other hand, teriparatide is known to be involved in calcium homeostasis in the bodies and is one of the causations of nausea accompanying the administration of teriparatide (Non-Patent Publication 23). In addition, by repeatedly administering teriparatide, a high blood calcium level is maintained or enhanced by the physiological activity of such teriparatide, whereby consequently side effect risks such as hypercalcemia and hypercalciuria may be considered.

In the present invention, one embodiment includes a liquid pharmaceutical preparation in which a time course in a state of a plasma concentration of teriparatide or a salt thereof after subcutaneous administration of a unit dose having a specified threshold value or more is within a specified range, and examples include two embodiments for the specified threshold values.

Supposing that one is a specified threshold value a, and the other is a specified threshold value b, both of the specified threshold value a and the specified threshold value b are not particularly limited. The specified threshold value a is preferably 50 (pg/mL) or more, and can be 60 (pg/mL) or more or 80 (pg/mL) or more, and it is preferable that the upper limit of the specified threshold value a is 200 (pg/mL) or less, 150 (pg/mL) or less, or 120 (pg/mL) or less. Preferred examples of the specified threshold value a include preferably 100 (pg/mL). By having a time course in a state of the plasma Component 1 concentration of a specified threshold value a or more within a particular range, an increase in the blood calcium concentration accompanying administration of a unit dose is inhibited. The inhibition of an increase in the blood calcium concentration can contribute to reductions in development frequency of digestive system side effects and/or development risks of hypercalcemia/hypercalciuria.

Here, the specified range of the time course is not particularly limited, and the time course can be within 3 hours, and can be preferably less than 2.5 hours, less than 2.1 hours, less than 2.0 hours, less than 1.73 hours, less than 1.7 hours, less than 1.5 hours, and further less than 1.0 hour. The lower limit thereof is not particularly limited, and can be 0.5 hours or more, 0.7 hours or more, and further 0.8 hours or more. In particular, it is more preferable that the time course is less than 2.1 hours, from 0.7 to 2.1 hours, less than 1.7 hours, or from 0.7 to 1.7 hours.

The specified threshold value **b** is also not particularly limited as mentioned above, and is preferably 100 (pg/mL) or more, and can be 150 (pg/mL) or more, or 200 (pg/mL) or more, and it is preferable that the upper limit is 500 (pg/mL) or less, 400 (pg/mL) or less, or 300 (pg/mL) or less. Preferred examples of the specified threshold value **b** include preferably 250 (pg/mL). By having a time course in a state of the plasma Component 1 concentration of a specified threshold value **b** or more within a particular range, an excellent safety accompanying administration of a unit dose (in particular, safety of suppressing the frequencies of the development of digestive tract side effects) can be preferably shown.

The above time course is not particularly limited, and the time course can be less than 1.4 hours, and can be preferably less than 1.3 hours, less than 1.2 hours, less than 1.1 hours, less than 1.0 hour, and further less than 0.9 hours, less than 0.8 hours, or less than 0.7 hours. The lower limit thereof is not particularly limited, and the time course can be 0.0 or more, and further 0.1 hours or more. In particular, it is more preferable that the time course is less than 0.8 hours and 0.1 hours or more.

When a liquid pharmaceutical preparation of the present invention is subcutaneously administered in a unit dose, in general, it is considered that the plasma concentration of Component 1 also tends to increase along with the increase in a unit dose of Component 1. Therefore, in order that a time course in a state of the plasma Component 1 concentration of a specified threshold value or more is within a specified range, the unit dose of the Component 1 is preferably properly regulated within the above range, and it is most preferable to regulate to 28.2 $\mu$g, in terms of teriparatide.

Considerations can be taken on a possibility that the ionization of the Component 1 contained in the liquid pharmaceutical preparation of the present invention influences the absorption of the Component 1 when subcutaneously administered. Therefore, for the purpose of regulating a time course in a state of the plasma Component 1 concentration of a specified threshold value or more, the Component 1 contained in the liquid pharmaceutical preparation of the present invention can be formed into a salt of teriparatide and one or more volatile organic acids, or a pH of the liquid pharmaceutical preparation of the present invention can be properly adjusted in reference to Examples set forth below. Also, for the same purposes as above, an additive of the liquid pharmaceutical preparation of the present invention can be properly selected in reference to Examples set forth below.

In addition, since the time course from the time point reaching the above specified threshold value of the Component 1 to the time point below the same value is defined as the above time course, the concentration of the Component 1 contained in the liquid pharmaceutical preparation of the present invention is preferably properly adjusted within the above range, and the concentration can be, for example, from 80 to 240 $\mu$g/mL, from 100 to 200 $\mu$g/mL, from 109 to 190 $\mu$g/mL, or from 120 to 160 $\mu$g/mL.

In a case of an embodiment in which two specified threshold values of the Component 1 are present (specified threshold values **a, b,** wherein **b > a),** by making $T_{max}$ of the Component 1 smaller, a time course from the time point reaching a specified threshold value **a** to the time point below the same value (time course **a)** could be even more shortened; however, by exceedingly making $T_{max}$ smaller, the time course from the time point reaching a specified threshold value **b** to the time point below the same value (time course **b)** may be lengthened. Therefore, in such a case, it is preferable that both of the time course **a** and the time course **b** are shortened in a good balance, to make the safety in administration of a unit dose favorable, and more specifically, for example, it is desired that the concentration of the Component 1 contained in the liquid pharmaceutical preparation of the present invention is adjusted to the above concentration range, or that $T_{max}$ of the Component 1 is adjusted within the above time range.

The absorption rate constant (Ka) of the Component 1 obtained when the liquid pharmaceutical preparation is subcutaneously administered to a subject to be administered becomes large by increasing the concentration of the Component 1 contained in the liquid pharmaceutical preparation (Non-Patent Publication 26). As Ka of the Component 1 is increased, $T_{max}$ of the Component 1 is shortened, whereby consequently the slope of the elimination phase of the plasma Component 1 concentration can be large (specifically, since the flip-flop phenomenon is likely to be eliminated, the slope of the elimination phase can approximate an elimination rate constant). The shortening of $T_{max}$ of the Component 1 and the increase in the slope of the elimination phase of the plasma Component 1 concentration can shorten the time course from the time point of reaching a specified threshold value mentioned above of the Component 1 to the time point of below the same value.

In the present invention, in a case where a subject to be administered is human, as to the human to which a liquid pharmaceutical preparation of the present invention is administered, it is preferable that the gender is preferably female, that the age is 45 years old or higher (preferably 50 years old or higher), and that the body weight is from 42 to 62 kg (preferably from 45 to 60 kg), respectively.

In addition, in the present invention, in a case where a subject to be administered is human, for the purpose of regulating a time course in a state of a plasma Component 1 concentration having a specified threshold value or higher, human to which a liquid pharmaceutical preparation of the present invention is administered can be, for example, postmenopausal women (Non-Patent Publication 27).

Alternatively, in the present invention, in a case where a subject to be administered is human, a dosage can also be properly regulated by the judgments of the physicians or the like in accordance with the body weight or the like of human to which a liquid pharmaceutical preparation of the present invention is administered.

The plasma Component 1 concentration obtained when a liquid pharmaceutical preparation of the present invention is subcutaneously administered in a unit dose can be confirmed by a measurement method that itself is known (see, FIG. 6). The site of subcutaneous administration is preferably, but not particularly limited to, sites that have smaller distributions of nerves or blood vessels, larger subcutaneous fats, and no bones. Such sites preferably include abdominal parts, upper arm parts, femur parts, and hip parts, and abdominal parts are most preferred.

When the plasma Component 1 concentration is measured, it is preferable to secure a sufficient number of measurement time points. As shown in various evaluation procedures in Examples set forth below, for example, it is preferable that blood samples are collected before the administration, and after 5, 15, 30, and 45 minutes, and after 1, 1.5, 2, 3, 4, and 6 hours of administration to measure a plasma concentration of the Component 1.

### (7) pH. Additives, and Buffer:

The pH of a liquid pharmaceutical preparation according to the present invention preferably includes, but not particularly limited to, as follows. Specifically, it is preferable that the pH of the liquid pharmaceutical preparation is, for example, 3.5 or more, 4.0 or more, exceeding 4.0, 4.2 or more, or 4.4 or more. It is preferable that the pH of the liquid pharmaceutical preparation is, for example, 6.0 or less, 5.5 or less, 5.0 or less, less than 5.0, 4.9 or less, or 4.8 or less. In particular, it is preferable that the pH is preferably 5.0 or less, and further preferably 4.0 or more and 5.0 or less, 4.0 or more and less than 5.0, 4.2 or more and less than 5.0, and it is most preferable that the pH is 4.4 or more and 4.9 or less. Excellent stability (for example, formation inhibition of deamidation product or the formation of cleavage products(31-34) of the Component 1, and the like) and/or pharmacokinetics can be efficiently obtained by having a pH of the present preparation of the above range.

In addition, a liquid pharmaceutical preparation of the present invention can contain various additives. The additives include, for example, solubilizers, stabilizers, isotonic agents, pH adjusting agents, anticorrosives (preservatives), and the like. Examples of the additives include, for example, sodium chloride, D-mannitol, sucrose, and L-methionine. The pH adjusting agent includes, for example, hydrochloric acid and sodium hydroxide.

Also, a liquid pharmaceutical preparation of the present invention may contain a buffer which is generally used in the pharmaceutical fields. Alternatively, the preparation of the present invention may be a liquid pharmaceutical preparation which substantially does not contain a buffer. In particular, since the preparation is a liquid pharmaceutical preparation substantially not containing an acetate buffer, excellent pharmacokinetics can be efficiently obtained.

In a case where a liquid pharmaceutical preparation of the present invention contains at least one or more members of inorganic salts and/or organic salts, a concentration thereof is not particularly limited, and the concentration is preferably 2 mg/mL or more, and more preferably 3 mg/mL or more, and in particular even more preferably 5.5 mg/mL or more. On the other hand, the concentration is preferably 25 mg/mL or less, and in particular more preferably 11 mg/mL or less.

In a case where a liquid pharmaceutical preparation of the present invention contains at least one or more members of inorganic salts and/or organic salts, a mass ratio thereof to teriparatide or a salt thereof (a mass ratio of Component 1 : Component 2) is not particularly limited, and the lower limit is, for example, preferably 1:5 or more, and even more preferably 1:10 or more, or 1:15 or more, and in particular more preferably 1:20 or more, and most preferably 1:35 or more. On the other hand, the upper limit is, for example, preferably 1:500 or less, more preferably 1:300 or less, and most preferably 1:80 or less.

The pH of a liquid pharmaceutical preparation of the present invention can be adjusted with methods that themselves are known, for example, a buffer or a pH adjusting agent.

In addition, one embodiment of a liquid pharmaceutical preparation of the present invention includes a liquid pharmaceutical preparation which contains 28.2 $\mu$g or 56.5 $\mu$g of teriparatide acetate in a unit dose, in terms of teriparatide, further excluding a freeze-dried preparation containing sodium chloride and purified white sugar. Further, one embodiment of a liquid pharmaceutical preparation of the present invention includes a liquid pharmaceutical preparation excluding a liquid pharmaceutical preparation which contains glacial acetic acid, sodium acetate (which may be in the form of anhydride), and D-mannitol, wherein its pH is from 3.8 to 4.5 (for example, a pH of 4.1). Alternatively, one embodiment of a liquid pharmaceutical preparation of the present invention includes a liquid pharmaceutical preparation excluding a freeze-dried preparation which contains 28.2 $\mu$g or 56.5 $\mu$g of teriparatide acetate in a unit dose, in terms of teriparatide. In addition, one embodiment of a liquid pharmaceutical preparation of the present invention includes a liquid pharmaceutical preparation excluding a freeze-dried preparation containing Component 1 and a monosaccharide (for example, mannitol, glucose, sorbitol, inositol). Alternatively, one embodiment of a liquid pharmaceutical preparation of the invention includes a liquid pharmaceutical preparation excluding a liquid pharmaceutical preparation containing Component 1 and xylitol.

(8) Freeze-Drying:

A liquid pharmaceutical preparation of the present invention may embrace embodiments of liquid pharmaceutical preparations reconstituted from freeze-dried preparations, or the liquid pharmaceutical preparation may not be liquid pharmaceutical preparations which are reconstituted from freeze-dried preparations. Conventionally, it has been known that a freeze-dried preparation containing teriparatide or a salt thereof is dissolved (or redissolved) with physiological saline or the like upon use to prepare a liquid pharmaceutical preparation. A liquid pharmaceutical preparation of the present invention may be a redissolved product of a freeze-dried preparation described above (prepared product upon use), or may be a preparation without undergoing a freeze-dried preparation (previously liquefied preparation). In the present invention, a preparation having excellent pharmacokinetics can be provided without going through the freeze-drying preparation.

(9) Pharmacokinetics:

In one embodiment of the liquid pharmaceutical preparation for subcutaneous administration of the present invention, the $\alpha$-helix content ratio of teriparatide or a salt thereof (Component 1) is within a specified range (for example, 13.0% or more). In addition, in one embodiment of the liquid pharmaceutical preparation for subcutaneous administration of the present invention, the number of amino acid residues that form an $\alpha$-helix is within a specified range (for example, 4.5 or more). In the subcutaneous liquid pharmaceutical preparation described above, excellent pharmacokinetics are obtained.

When a liquid pharmaceutical preparation is administered to a mammal such as human or a monkey, to what extent the preparation reaches and acts on the systemic circulation blood is an important problem. In general, when a liquid pharmaceutical preparation is intravenously administered, the drug in the above preparation is utilized nearly perfectly in live bodies, and when a liquid pharmaceutical preparation is administered by non-intravenous administration (oral, rectal, transdermal, or subcutaneous, or the like), not all reach the circulation blood. As an index of measuring an amount that reaches the systemic circulation blood, AUC (area under the plasma concentration versus(-) time curve) is employed in many cases. In addition, bioavailability of a drug may be evaluated as (absolute) bioavailability rate (%) which is a ratio of the AUC obtained by the non-intravenous administration to the AUC obtained by the intravenous administration. It is important to improve pharmacokinetic parameters such as AUC by non-intravenous administration and bioavailability rate, from the viewpoint of increasing therapeutic effects, the safety or the like offered by the drug.

The pharmacokinetics of a liquid pharmaceutical preparation can be evaluated using various pharmacokinetic parameters as indices. Examples of the pharmacokinetic parameter preferably include a time to the maximum plasma concentration ($T_{max}$), the maximum plasma concentration (Cmax), an area under the plasma concentration versus(-) time curve (AUC), bioavailability rate (%), and the like. The AUC includes, but not particularly limited to, for example, $AUC_{inf}$ (an area under the plasma concentration versus(-) time curve until infinitesimal time), $AUC_{last}$ (an area under the plasma concentration versus(-) time curve until the last observation time), and $AUC\tau$ (an area under the plasma concentration versus(-) time curve from time 0 to an administration interval time $\tau$) obtained during repetitive administrations in unit-dose intervals, and the like.

During the evaluation of the pharmacokinetic parameters, the site of administration is preferably, but not particularly limited to, sites that have smaller distributions of nerves or blood vessels, larger subcutaneous fats, and no bones. Such sites preferably include abdominal parts, upper arm parts, femur parts, and hip parts, and abdominal parts are most preferred.

The method of calculating a pharmacokinetic parameter is not particularly limited, and the parameters can be calculated by using any of analyses independent of pharmacokinetic models and analysis methods dependent on pharmacokinetic models (for example, 1-compartment model) (Non-Patent Publication 6). However, parameters are preferably calculated by analysis methods independent of pharmacokinetic models, specifically NCA (Non Compartmental Analysis). The method of calculating AUC in accordance with NCA includes a linear trapezoidal rule and a logarithmic linear trapezoidal rule. For example, AUC can also be calculated by using a linear trapezoidal rule in an absorption phase up to a time to the maximum plasma concentration ($T_{max}$), and a logarithmic linear trapezoidal rule in an elimination phase on or after $T_{max}$.

When a pharmacokinetic parameter is calculated, it is preferable to secure a sufficient number of measurement time points. As shown in various evaluation procedures in Examples set forth below, for example, it is preferable that blood samples are collected before the administration, and after 5, 15, 30, and 45 minutes, and after 1, 1.5, 2, 3, 4, and 6 hours of administration to measure a plasma concentration of teriparatide or a salt.

In order to calculate a pharmacokinetic parameter of a liquid pharmaceutical preparation, it is preferable to secure a sufficient number of cases. Each of the pharmacokinetic parameters may be a mean obtained by dividing the sum of numerical values shown by each cases by the number of cases, or in the alternative, the numerical values shown by each case may be placed in numerical order to define as its median positioned in its center. In order to obtain pharmacokinetic parameters of plural kinds of liquid pharmaceutical preparations, group comparison tests and crossover tests can be employed. Since teriparatide can be relatively easily washed out and the number of cases can be made compact, it is preferable to apply crossover tests, for the purpose of obtaining pharmacokinetic parameters of plural kinds of liquid pharmaceutical preparations.

As an index of the pharmacokinetics, the absolute bioavailability rate (%) of Component 1 can be calculated by, for example, the following formula.

[Math Formula 3]

$$\text{Absolute Bioavailability Rate (\%) of Component 1} = \frac{\left\{\begin{array}{l}\text{AUC}_{inf} \text{ of Component 1 Obtained} \\ \text{by Subcutaneous Administration}\end{array}\right\} \times \left\{\begin{array}{l}\text{Dosage of Component 1 by} \\ \text{Intravenous Administration}\end{array}\right\}}{\left\{\begin{array}{l}\text{AUC}_{inf} \text{ of Component 1 Obtained} \\ \text{by Intravenous Administration}\end{array}\right\} \times \left\{\begin{array}{l}\text{Dosage of Component 1 by} \\ \text{Subcutaneous Administration}\end{array}\right\}} \times 100 \ (\%)$$

Depending upon the measurement errors or the like of the $\text{AUC}_{inf}$, an absolute bioavailability rate (%) exceeding 100%, which is a theoretical upper limit, may be obtained. Examples of the absolute bioavailability rate (%) of Component 1 include, but not particularly limited to, as follows. Specifically, it is preferable that the absolute bioavailability rate is, for example, 70% or more, 80% or more, 90% or more, 95% or more, 100% or more, and 110% or more. In addition, it is preferable that the upper limit is, for example, 180% or less, 160% or less, and 150% or less. In particular, the absolute bioavailability rate is preferably 90% or more and 160% or less, and most preferably 100% or more and 150% or less.

Examples of the $C_{max}$ of Component 1 include, but not particularly limited to, as follows. Specifically, it is preferable that $C_{max}$ is 230 (pg/mL) or more, and 240 (pg/mL) or more, and 250 (pg/mL) or more. In addition, it is preferable that the upper limit is, for example, 380 (pg/mL) or less, 360 (pg/mL) or less, and 350 (pg/mL) or less. In particular, the $C_{max}$ is preferably from 250 to 350 (pg/mL).

Examples of the $\text{AUC}_{last}$ of Component 1 include, but not particularly limited to, as follows. Specifically, it is preferable that the $\text{AUC}_{last}$ is 350 (hr•pg/mL) or more, 360 (hr•pg/mL) or more, 370 (hr•pg/mL) or more, 380 (hr•pg/mL) or more, and 390 (hr•pg/mL) or more. In addition, it is preferable that the upper limit is, for example, 600 (hr•pg/mL) or less, 580 (hr•pg/mL) or less, 570 (hr•pg/mL) or less, 550 (hr•pg/mL) or less, and 530 (hr•pg/mL) or less. In particular, the $\text{AUC}_{last}$ is preferably from 350 to 550 (hr•pg/mL).

Examples of the $\text{AUC}_{inf}$ of Component 1 include, but not particularly limited to, as follows. Specifically, it is preferable that the $\text{AUC}_{inf}$ is 380 (hr•pg/mL) or more, 390 (hr•pg/mL) or more, 400 (hr•pg/mL) or more, and 420 (hr•pg/mL) or more. In addition, it is preferable that the upper limit is, for example, 650 (hr•pg/mL) or less, 600 (hr•pg/mL) or less, 590 (hr•pg/mL) or less, 580 (hr•pg/mL) or less, and 560 (hr•pg/mL) or less. In particular, the $\text{AUC}_{inf}$ is preferably from 400 to 600 (hr•pg/mL).

It is preferable that at least one or more members of the absolute bioavailability rate (%), $T_{max}$, $C_{max}$, $\text{AUC}_{last}$, and $\text{AUC}_{inf}$ of Component 1 are within the ranges defined above.

Here, it is still unknown that the above epoch-making results are shown by what mechanisms.

In the aspect of pharmacokinetics, for example, an antibody preparation for subcutaneous administration to be used in clinical situations only has bioavailability of roughly from 50 to 60%, and the causations for possibly inducing such a low bioavailability are reportedly due to diversified matters such as the electric charges or hydrophobicity shown by the protein in the preparation, the additive components in the preparation, and the dosage and administration depth, and the positively charged antibody being adsorbed to subcutaneous tissues (Non-Patent Publication 7). However, the influences which the secondary structure of the antibody in the preparation gives to bioavailability are neither disclosed nor suggested at all. In a different report, it is disclosed that when a drug solution prepared by dissolving a teriparatide acetate freeze-dried preparation (additives being purified white sugar and sodium chloride) in physiological saline (pH 5.0 to 7.0) is subcutaneously administered, a maximum blood concentration is reached in about 35 to 50 minutes or so, and an absolute bioavailability rate calculated from $\text{AUC}_{inf}$ is nearly 100% ("Section of [Pharmacokinetics] 2. Bioavailability Rate" in Non-Patent Publication 1); however, the publication does not suggest at all on the influences of the secondary structure of teriparatide acetate in the drug solution on bioavailability.

On the other hand, in the aspect of the secondary structure of teriparatide, it is reported that, for example, teriparatide is mainly flexible and stretching in an aqueous solution, with an exception to the existence of a partial structure which is not random at positions 20 to 24 (Arg-Val-Glu-Trp-Leu) from the N-terminal, that a secondary structure in accordance with a two-dimensional NMR measurement is hardly observed, and the like (Non-Patent Publication 12). However, in this publication, the influences of the secondary structure of teriparatide on pharmacokinetics such as absorption, metabolism, and elimination are not suggested in any manner.

As described above, it is difficult to discuss the influences of the secondary structure of teriparatide on pharmacokinetics only on the bases of conventional reports. In view of the above situations, the present inventors have discussed on the matter that the above epoch-making results can be acquired by what mechanisms as follows.

Skin serves important roles of isolating inside the body from the outside to maintain homeostasis of human bodies, so that the skin has various functions in order to serve the roles, and has complicated structures for realizing those roles. If the skin is observed from a cross section, it can be seen that the skin roughly has a three-layer structure of epidermal, dermal, and subcutaneous tissues. The subcutaneous tissues are mainly adipose tissues, which play the roles of storage of neutral fats, thermal function, and a cushion from an outer force.

The constitution of a pharmaceutical preparation to be administered subcutaneously is different from the structure of a subcutaneous tissue to be administered, so that it is proposed that various stresses are probably applied to stability, dissolubility, and the functions of the preparations after subcutaneous administration, and during which a drug reaches blood vessels or lymphoducts (Non-Patent Publication 8). Here, as described in Non-Patent Publication 8, as examples of stresses, 1) steric structural hindrance in the extracellular matrix, electrostatic interactions, and specific interactions (Table 4), 2) influences of pH fluctuations before and after administration on protective actions of an additive each contained in a pharmaceutical preparation to a drug (pages 27 to 28), 3) aggregation of a drug and adsorption to subcutaneous tissues due to fast migration of an additive after administration (D of FIG. 5), 4) influences of pH fluctuations before and after administration on drug stability (page 29), 5) influences of temperature fluctuations near a drug before and after administration on drug absorbability (page 29), 6) influences of fluctuations of interstitial fluid pressure or colloidal osmotic pressure by administration on drug stability (pages 29 and 30), and the like have been known.

The present inventors consider, as one theory, that the $\alpha$-helix content in teriparatide or a salt thereof is involved in at least one of the above stresses, without binding the present invention thereto.

The involvement as mentioned herein is not particularly limited, so long as teriparatide or a salt thereof which is subcutaneously administered has the mechanism of showing excellent pharmacokinetics. For example, as one technical idea, an embodiment in which $\alpha$-helix or an amount thereof in teriparatide or a salt thereof increases its bioavailability rate (%) by 1) improving permeability of vascular endothelium can be presented.

The vascular endothelial cells are cells located in an innermost layer of the blood vessels running around systemically, which play important roles of adhesion of inflammatory cells to blood vessels, vascular permeability, regulation of coagulation and fibrinolytic systems, and the like. On the other hand, $\alpha$-helix of peptides is known to be greatly involved in the membrane permeation of peptides (Non-Patent Publication 28).

Therefore, as one theory, in a case where a certain degree or higher of $\alpha$-helix is present in teriparatide or a salt thereof which is subcutaneously administered, the membrane permeability of the vascular endothelial cells of the peptides is increased as compared to a case where in the absence thereof, so that the migration to blood is increased, whereby consequently a mechanism in which bioavailability rate (%) is increased can also be considered.

In addition, for example, as one technical idea, an embodiment in which the $\alpha$-helix or an amount thereof in teriparatide or a salt thereof directly or indirectly inhibits 2) various hindrances or interactions in the extracellular matrices, thereby increasing the bioavailability rate (%) can be presented.

The extracellular matrix is a supramolecular structure which exists outside the cells, which has a backbone role and at the same time provides scaffold in cell adhesion, which is involved in signaling or the like. The extracellular matrix is constituted by structural proteins (collagens or the like), proteoglycans, and the like. The proteoglycan is a complex in which glycosaminoglycan (may be referred to as GAG in some cases) is covalently bonded to a protein that serves as a core. Examples of the GAG include chondroitin sulfate, hyaluronic acid, heparin, and the like. It is known that the collagen or GAG can cause specific interactions with a drug which is subcutaneously administered (Non-Patent Publication 8).

On the other hand, a parathyroid hormone PTH(1-84) has been known that $\alpha$-helix is induced by an interaction with heparin or various polyanionic materials (Non-Patent Publication 29). It is considered that the interactions between the GAG and various proteins regulate various biological phenomena in stages, and the like, and heparin, when interacted with a heparin-binding protein, prioritizes the same protein having a native structure. On the bases of the above, PTH(1-84) caused a structural change of $\alpha$-helix by an interaction with the GAG, and a model in which PTH(1-84) which undergoes a structural change as described above binds to a receptor is provided (Non-Patent Publication 29).

Therefore, as one theory, in a case where a certain degree or higher of $\alpha$-helix is present in teriparatide or a salt thereof which is subcutaneously administered, the interaction with the GAG is lessened or weakened, as compared to a case in the absence thereof, whereby consequently a mechanism in which bioavailability rate (%) is increased can also be considered. The mechanisms of the influences of the $\alpha$-helix content on the interactions between teriparatide or a salt thereof and the GAG are not particularly limited, and, for example, it can be considered as changes in balance between polarity and non-polarity in teriparatide or a salt thereof.

Conventionally, it is reported that teriparatide in the aqueous solution is mainly flexible and stretching (Non-Patent Publication 12), so that the present inventors have discussed that there is a high possibility that teriparatide does not have a significant difference in a tertiary structure.

Further, at the present time, no distinct relationships were found between the zeta potential of teriparatide in the aqueous solution and the pharmacokinetics. In view of above, the inventors consider that the certainties of the relationships between the $\alpha$-helix content in teriparatide in the aqueous solution or the number of amino acid residues that form $\alpha$-helix and the pharmacokinetics now are ascertained.

In teriparatide, the amino acid residues that form $\alpha$-helix may be any one of the positions 1 to 34 from the N-terminal. The amino acid residues may be, but not particularly limited to, for example, the positions 3 to 12, the positions 17 to 26, and the like. These amino acid residues are more likely to form a helical structure. For this reason, in the preparation of the present invention, at least one of the number of the amino acid residues may form $\alpha$-helix.

In particular, of these amino acid residues (the positions 3 to 12, the positions 17 to 26), amino acid residues having 4 or more in average (for example, 4.2 or more, 4.4 or more, 4.42 or more, 4.5 or more, 4.59 or more, 4.6 or more, 4.69 or more, or 4.7 or more) may form $\alpha$-helix. In addition, of these amino acid residues (the positions 3 to 12, the positions 17 to 26), amino acid residues having 20 or less in average (for example, 18 or less, 16 or less, 15 or less, 12 or less, 11 or less, 10 or less, 9 or less, 8 or less, 7 or less, 6.8 or less, 6.5 or less, 6.1 or less, 5.5 or less, 5.44 or less, 5.4 or less, or 5.37 or less) may form $\alpha$-helix.

In addition, among the amino acid residues, at least one amino acid residue selected from the position 13 (lysine residue), the position 14 (histidine residue), and the position 27 (lysine residue) may form $\alpha$-helix. All of these residues are basic amino acid residues, so that it is assumed that the residues would be positively charged when administered to subcutaneous tissues.

These amino acid residues seem to be relatively strongly influenced by any of the stresses mentioned above, and the formation of $\alpha$-helix by these amino acid residues is allowed to obtain excellent pharmacokinetics efficiently.

(10) Safety:

In one embodiment of a liquid pharmaceutical preparation of the present invention, a unit dose of teriparatide or a salt thereof is a specified amount (for example, 28.2 $\mu$g). Alternatively, in one embodiment of a liquid pharmaceutical preparation of the present invention, a time to the maximum plasma concentration ($T_{max}$) of teriparatide or a salt thereof obtained by administration of a unit dose is within a specified range (for example, less than 0.7 hours). Alternatively, in one embodiment of a liquid pharmaceutical preparation for subcutaneous administration of the present invention, a time course in a state of a plasma concentration of teriparatide or a salt thereof obtained by administration of a unit dose having a specified threshold value (for example, 100 pg/mL, or 250 pg/mL) or more is within a specified range (for example, less than 2.1 hours, or less than 1.0 hour). In these subcutaneous liquid pharmaceutical preparations, excellent safety is obtained.

Here, the safety embraces all the adverse events which take place unfavorably in medical situations and any side effects of which cause-effect relationships between the adverse events and the drug cannot be denied.

Serious adverse events include death, impairments, and the like, and the safety in the present invention embraces, but not limited thereto, all sorts of risks that can influence the evaluations in the relationships with the efficacy of the pharmaceuticals (benefits).

The kinds and the degrees of safety are not particularly limited. Examples include, for example, impairments and unwanted symptoms that take place in skin and skin attachments, muscles and bones, central and peripheral nerves, autonomic nerves, vision, olfaction, mentality, digestive tract, the liver and bile duct, metabolic and trophic impairments, endocrine, the heart and blood vessels, the respiratory system, blood cells and blood platelets, urinary organs, genital organs, system, or the like, and the intensities and frequencies thereof are not limited. Examples include preferably digestive tract side effects and blood pressure lowering risks, among which examples of the frequencies of nausea, vomiting, and gag are most preferred.

The pharmaceutical is repeatedly and continuously used over a period of time as a therapeutic agent for lifestyle diseases in many cases, and the continuality of the therapy is important to obtain a favorable treatment. However, if a medicament is repeatedly administered, a trough value is increased, so that side effects may be stronger in some cases, and a treatment drop-out caused by increase in such side effects can cause bad influences on the treatment.

Alternatively, side effects may be frequently or strongly developed by transiently increasing a blood concentration of a pharmaceutical every time of administration of the pharmaceutical, and in such a case, unwanted situations such as treatment drop-out can be consequently caused.

As described above, upon the utilization of the pharmaceutical, it is desired that the safety is considered every time of its use and over the period of continuous use. In other words, it is preferable that the medicament is provided with the safety in both aspects of the safety accompanying administration of a unit dose and the safety accompanying repeated continuous administration.

It is preferable that in a liquid pharmaceutical composition of the present invention, safety accompanying administration of a unit dose is improved, as compared to the conventional pharmaceutical preparations containing teriparatide. Examples of improvement in safety accompanying administration of a unit dose are not limited thereto, and examples preferably include reduction in digestive tract side effect frequency and/or blood pressure lowering risks accompanying administration of a unit dose.

(11) Properties and the like:

A liquid pharmaceutical preparation of the present invention is preferably colorless and transparent at least during its production, and its osmotic pressure ratio to physiological saline can be about 1 (for example, from 1.0 to 1.4).

2. Method for Producing Liquid Pharmaceutical Preparation:

A liquid pharmaceutical preparation of the present invention is producible in accordance by various methods that themselves are known. Usually, various components mentioned above that constitute a liquid pharmaceutical preparation of the present invention are appropriately selected, which may be mixed with a proper solvent to dissolve.

In a case where a liquid pharmaceutical preparation for subcutaneous administration of the present invention is produced, it is preferable to make it an aqueous liquid pharmaceutical preparation. In the case of an aqueous liquid pharmaceutical preparation, it is preferable that it is subjected to a sterile treatment before administration. When the aseptic processing is adopted as the aseptic treatment, a liquid pharmaceutical preparation can be prepared by dissolving each of weighed raw materials in a water for injection or the like, and subjecting a dissolved solution to filtration sterilization. The water for injection is generally understood as sterile purified water which is compatible to an endotoxin test, and a water for injection produced by distillation method may be also called a distilled water for injection.

This liquid pharmaceutical preparation for injection is further packed and sealed in a washed and sterile treated container, and being subjected to examination, packaging or the like, whereby an injection comprising a liquid pharmaceutical preparation for injection packed therein can be produced. Examples of the container as used herein include, for examples, ampules, vials, pre-filled syringes, bags, and the like. The materials of the container include, but not particularly limited to, glass and plastics. Examples of the material for the container preferably include plastics, from the viewpoint of strength, easiness in handling, safety, and the like.

3. Method for Improving Pharmacokinetic Parameter:

The present invention, as one embodiment, provides a method, when a liquid pharmaceutical preparation containing Component 1 is subcutaneously administered, for improving a pharmacokinetic parameter of Component 1 shown by the above preparation, including adjusting (increasing or the like) an $\alpha$-helix content ratio and/or the number of amino acid residues that form $\alpha$-helix in Component 1.

This method can be carried out by, for example, sequentially carrying out the following steps.

step 1) preparing a liquid pharmaceutical preparation containing Component 1 so that an $\alpha$-helix content ratio of the Component 1 is within a specified range defined above (for example, 13.0% or more) and/or that the number of amino acid residues that form $\alpha$-helix in Component 1 is within a specified range defined above (for example, 4.5 or more);

step 2) administering the liquid pharmaceutical preparation to human subcutaneously and collecting blood samples from human before administration and at plural time points after administration;

step 3) measuring a concentration of the Component 1 contained in the blood samples at each time point;

step 4) calculating a numerical value A of a certain pharmacokinetic parameter a from Component 1 concentration at each time point;

step 5) comparing a numerical value B of a pharmacokinetic parameter a obtained when a liquid pharmaceutical preparation containing Component 1 in which an $\alpha$-helix content ratio in Component 1 and/or the number of amino acid residues that form $\alpha$-helix in Component 1 is outside the specified range is administered to human subcutaneously with the numerical value A, and judging whether or not the numerical value A is more excellent than the numerical value B.

In a case where the pharmacokinetic parameter is an absolute bioavailability rate (%) of Component 1, an increase of its numerical value means improvements of pharmacokinetic parameters. In a case where the pharmacokinetic parameter is $AUC_{last}$ or $AUC_{inf}$ of the Component 1, the increase of its numerical value means the improvement in pharmacokinetic parameter.

In addition, the present invention provides, as one embodiment, a method, when a liquid pharmaceutical preparation containing teriparatide or a salt thereof as Component 1 is subcutaneously administered, for improving a pharmacokinetic parameter of Component 1 shown by the above preparation, characterized in that the method includes at least one member of 1) having a unit dose of Component 1 within a specified amount defined above (for example, 28.2

μg), 2) having a Component 1 concentration within a specified range (for example, from 120 to 160 μg/mL), 3) making Component 1 a salt with one or more volatile organic acids, 4) adjusting a pH of a liquid pharmaceutical preparation, and 5) properly containing additives in the preparation. Here, the improvement of the pharmacokinetic parameter can be confirmed by measuring whether or not $T_{max}$ of the Component 1 is within the above defined range (for example, from 0.2 to 0.7 (hr)).

4. Method for Controlling Quality:

The present invention provides, as one embodiment, a method for controlling quality of a liquid pharmaceutical preparation for subcutaneous administration containing Component 1, including measuring an α-helix content ratio of the Component 1 and/or the number of amino acid residues that form α-helix in Component 1 in the liquid pharmaceutical preparation, comparing the obtained measurement values of the α-helix content ratio and/or the number of amino acid residues that form α-helix in Component 1 with a previously determined standard values, and judging that quality of the liquid pharmaceutical preparation is maintained in a case when the above measurement values are equal to or higher than the above standard values.

Here, the previously determined standard value is the specified range lower limit of the α-helix content ratio of the Component 1 mentioned above (for example, 13.0% or more).

In addition, the value to be compared with the standard value can also be the number of α-helical structure form residues, and the previously determined standard value in that instance is defined as the lower limit of the range of the residues that form α-helical structure in the Component 1 mentioned above (for example, 4.5 or more).

Alternatively, the value to be compared with the standard value can be an average residue molar ellipticity $[\theta]$ in accordance with the circular dichroism (CD) spectroscopy (measurement wavelength: 222 nm), and the previously determined standard value in that instance is an upper limit of the range of the average residue molar ellipticity $[\theta]$ as determined by the above circular dichroism spectroscopy (for example, -6300 or less).

Here, the quality of a liquid pharmaceutical preparation is a pharmacokinetic parameter obtained, for example, when a liquid pharmaceutical preparation is administered subcutaneously in a unit dose. Examples of the pharmacokinetic parameter preferably include absolute bioavailability rate (%) of the Component 1, $AUC_{last}$, $AUC_{inf}$, and the like.

EXAMPLES

The present invention will be explained more specifically by means of Examples. However, the present invention is not intended to be bound to the following Examples, and the present invention can be carried out in any embodiments within the scope that does not depart from the spirit of the present invention.

Here, in the following Examples, the term "formulation" may be expressed as a word corresponding to "a liquid pharmaceutical preparation" of the present invention.

Example 1 (Preparation of Liquid Pharmaceutical Preparations):

(1) Preparation of Liquid Pharmaceutical Preparations Subjected to Pharmacokinetic Tests in Monkeys:

Formulations A to H were prepared in accordance with the following Tables 1 and 2. Each of these formulations is nearly the identical formulation as Formulations A to H of "(2) Preparation of Liquid Pharmaceutical Preparations Subjected to Pharmacokinetic Tests in Human" given later, from the viewpoint of their components.

Formulations A to D were prepared in accordance with the following Table 1.

A specific preparation method for each formulation is as follows. First, each additive solution listed in the column of "Additives" in the table was mixed and its volume was adjusted to about 46 mL with a water for injection. Thereafter, 2.5 mL of a teriparatide acetate solution (2820 μg/mL in terms of teriparatide) was added to a mixed solution, to prepare about 48.5 mL of a drug solution a. Here, the solvent for each of the additive solution and the teriparatide acetate solution was a water for injection. Further, hydrochloric acid was added to the drug solution a to adjust its pH to that listed in the column of "pH" in the table, and a formulation of which entire volume was adjusted to 50 mL with a water for injection was prepared.

Each formulation was subjected to filtration sterilization, and a sterile formulation was filled in plastic vials in an amount of 1.5 mL each, to produce plastic vials filled with each formulation, to be subjected to pharmacokinetic tests in monkeys.

The components of each formulation are as listed in the column of "Final Content."

[Table 1]

Table 1

| Formulation | Additives | pH | Final Content |
|---|---|---|---|
| Formulation A | 50 mg/mL Sodium chloride: 8.25 mL<br>5 mg/mL L-Methionine: 1.0 mL<br>200 mg/mL Purified white sugar: 6.25 mL | 4.6 | Teriparatide: 141 $\mu$g/mL<br>Sodium chloride: 8.25 mg/mL<br>L-Methionine: 0.1 mg/mL<br>Purified white sugar: 25 mg/mL<br>Hydrochloric acid: 0.14 mM |
| Formulation B | 50 mg/mL Sodium chloride: 10.75 mL<br>5 mg/mL L-Methionine: 1.0 mL | 4.6 | Teriparatide: 141 $\mu$g/mL<br>Sodium chloride: 10.75 mg/mL<br>L-Methionine: 0.1 mg/mL<br>Hydrochloric acid: 0.14 mM |
| Formulation C | 100 mg/mL D-Mannitol: 28.5 mL | 4.1 | Teriparatide: 141 $\mu$g/mL<br>D-Mannitol: 57 mg/mL<br>Hydrochloric acid: 0.22 mM |
| Formulation D | 5 mg/mL L-Methionine: 1.0 mL<br>200 mg/mL Purified white sugar: 25 mL | 4.1 | Teriparatide: 141 $\mu$g/mL<br>L-Methionine: 0.1 mg/mL<br>Purified white sugar: 100 mg/mL<br>Hydrochloric acid: 0.22 mM |

Further, Formulations E to H were prepared in accordance with the following Table 2.

A specific preparation method for each formulation is as follows. First, each additive listed in the column of "Additives" in the table was mixed together with a water for injection to make into a total volume of 3000 mL. Thereafter, teriparatide acetate (282 mg in terms of teriparatide) was added to 1600 mL of the mixed solution to dissolve, to prepare a drug solution a. Further, a diluted hydrochloric acid was added to the drug solution a to adjust its pH to that listed in the column of "pH" in the table, and a total volume was then adjusted to 2,000 mL with a water for injection, to prepare a preparation.

Each formulation was subjected to filtration sterilization, and a sterile formulation was filled in plastic vials in an amount of 1.5 mL each, to produce plastic vials filled with each formulation, to be subjected to pharmacokinetic tests in monkeys.

The components of each formulation are as listed in the column of "Final Content."

[Table 2]

Table 2

| Formulation | Additives | pH | Final Content |
|---|---|---|---|
| Formulation E | D-Mannitol: 90.0 g<br>Sodium chloride: 16.5 g<br>L-Methionine: 105 mg | 4.6 | Teriparatide: 141 $\mu$g/mL<br>D-Mannitol: 30 mg/mL<br>Sodium chloride: 5.5 mg/mL<br>L-Methionine: 35 $\mu$g/mL<br>Hydrochloric acid: 0.13 mM |
| Formulation F | D-Mannitol: 90.0 g<br>Sodium chloride: 16.5 g<br>L-Methionine: 300 mg | 4.6 | Teriparatide: 141 $\mu$g/mL<br>D-Mannitol: 30 mg/mL<br>Sodium chloride: 5.5 mg/mL<br>L-Methionine: 0.1 mg/mL<br>Hydrochloric acid: 0.1385 mM |

(continued)

| Formulation | Additives | pH | Final Content |
|---|---|---|---|
| Formulation G | D-Mannitol: 135.0 g<br>Purified white sugar: 78.0 g<br>L-Methionine: 105 mg | 4.1 | Teriparatide: 141 μg/mL<br>D-Mannitol: 45 mg/mL<br>Purified white sugar: 26 mg/mL<br>L-Methionine: 35 μg/mL<br>Hydrochloric acid: 0.205 mM |
| Formulation H | Sodium chloride: 16.5 g<br>Purified white sugar: 156.0 g<br>L-Methionine: 300 mg | 4.6 | Teriparatide: 141 μg/mL<br>Sodium chloride: 5.5 mg/mL<br>Purified white sugar: 52 mg/mL<br>L-Methionine: 0.1 mg/mL<br>Hydrochloric acid: 0.1375 mM |

(2) Preparation of Liquid Pharmaceutical Preparations Subjected to Pharmacokinetic Tests in Human:

Formulations A to H were prepared in accordance with the following Table 3.

A specific preparation method for each formulation is as follows. First, each additive listed in the column of "Additives" in the table was mixed together with a water for injection (provided that L-methionine was a previously dissolved L-methionine solution), and teriparatide acetate (1425.6 mg in terms of teriparatide) was added thereto, to prepare a drug solution **a** in a total amount of 9.5 kg. Thereafter, a diluted hydrochloric acid was added to the drug solution **a** to adjust its pH to that listed in the column of "pH" in the table, and a formulation of which entire amount was adjusted to 10.10 kg with a water for injection was prepared.

Each formulation was subjected to filtration sterilization, and a sterile formulation was then filled in ampules in an amount of 2 mL each, to produce ampules filled with each formulation (formulation preparation), and subjected to pharmacokinetic tests in human. The formulation preparation is a preparation having a formulation volume of 0.2 mL, and containing 28.2 μg of teriparatide acetate in a unit dose, in terms of teriparatide.

The components of each formulation are as listed in the column of "Final Content."

[Table 3]

Table 3

| Formulation | Additives | pH | Final Content |
|---|---|---|---|
| Formulation A | Sodium chloride: 85.0 g<br>Purified white sugar: 250 g<br>L-Methionine: 1.00 g | 4.6 | Teriparatide: 141 μg/mL<br>Sodium chloride: 8.47 mg/mL<br>Purified white sugar: 24.9 mg/mL<br>L-Methionine: 0.1 mg/mL<br>Hydrochloric acid: 0.095 mM |
| Formulation B | Sodium chloride: 110.0 g<br>L-Methionine: 1.00 g | 4.6 | Teriparatide: 141 μg/mL<br>Sodium chloride: 11.1 mg/mL<br>L-Methionine: 0.1 mg/mL<br>Hydrochloric acid: 0.098 mM |
| Formulation C | D-Mannitol: 600 g | 4.1 | Teriparatide: 141 μg/mL<br>D-Mannitol: 60.2 mg/mL<br>Hydrochloric acid: 0.176 mM |
| Formulation D | Purified white sugar: 1040 g<br>L-Methionine: 1.00 g | 4.1 | Teriparatide: 141 μg/mL<br>Purified white sugar: 106.9 mg/mL<br>L-Methionine: 0.1 mg/mL<br>Hydrochloric acid: 0.195 mM |

(continued)

| Formulation | Additives | pH | Final Content |
|---|---|---|---|
| Formulation E | D-Mannitol: 300 g<br>Sodium chloride: 55.0 g<br>L-Methionine: 0.35 g | 4.6 | Teriparatide: 141 $\mu$g/mL<br>D-Mannitol: 30.1 mg/mL<br>Sodium chloride: 5.52 mg/mL<br>L-Methionine: 35.1 $\mu$g/mL<br>Hydrochloric acid: 0.1 mM |
| Formulation F | D-Mannitol: 300 g<br>Sodium chloride: 55.0 g<br>L-Methionine: 1.00 g | 4.6 | Teriparatide: 141 $\mu$g/mL<br>D-Mannitol: 30.1 mg/mL<br>Sodium chloride: 5.52 mg/mL<br>L-Methionine: 0.1 mg/mL<br>Hydrochloric acid: 0.1 mM |
| Formulation G | D-Mannitol: 450 g<br>Purified white sugar: 260 g<br>L-Methionine: 0.35 g | 4.1 | Teriparatide: 141 $\mu$g/mL<br>D-Mannitol: 45.6 mg/mL<br>Purified white sugar: 26.4 mg/mL<br>L-Methionine: 35.5 $\mu$g/mL<br>Hydrochloric acid: 0.198 mM |
| Formulation H | Sodium chloride: 55.0 g<br>Purified white sugar: 520 g<br>L-Methionine: 1.00 g | 4.6 | Teriparatide: 141 $\mu$g/mL<br>Sodium chloride: 5.57 mg/mL<br>Purified white sugar: 52.6 mg/mL<br>L-Methionine: 0.1 mg/mL<br>Hydrochloric acid: 0.101 mM |

(3) Preparation of Control Liquid Pharmaceutical Preparations:

(3-1) Preparation of Control Formulation 1:

To a commercially available teriparatide freeze-dried preparation ("Teribone for Subcutaneous Injection 56.5 $\mu$g" manufactured by ASAHI KASEI PHARMA CORPORATION; Non-Patent Publication 1) was added 0.45 mL of physiological saline adopted to Japanese Pharmacopoeia to dissolve, and a drug solution obtained was taken with a syringe in an amount of 0.2 mL to prepare Control Formulation 1, and a syringe filled with Control Formulation 1 was used as Control Formulation 1 Preparation. Here, Control Formulation 1 is a formulation having a volume of 0.2 mL and a teriparatide acetate concentration of 141 $\mu$g/mL, in terms of teriparatide, and containing 28.2 $\mu$g of teriparatide acetate in a unit dose, in terms of teriparatide.

(3-2) Preparation of Control Formulation 2:

To a commercially available teriparatide freeze-dried preparation ("Teribone for Subcutaneous Injection 56.5 $\mu$g" manufactured by ASAHI KASEI PHARMA CORPORATION; Non-Patent Publication 1) was added 1.0 mL of physiological saline adopted to Japanese Pharmacopoeia to dissolve, to prepare Control Formulation 2, and a syringe filled with Control Formulation 2 was used as Control Formulation 2 Preparation. Here, Control Formulation 2 is a formulation having a volume of 0.89 mL and a teriparatide acetate concentration of 63.5 $\mu$g/mL, in terms of teriparatide, containing 56.5 $\mu$g of teriparatide acetate in a unit dose, in terms of teriparatide.

(3-3) Preparation of Control Formulation 3:

Control Formulation 3 was prepared in accordance with the following Table 4.
A specific preparation method for each formulation is as follows. First, each additive listed in the column of "Additives" in the table was mixed together with a water for injection, to prepare a solution a in a total amount of 3000 g. Teriparatide acetate (352.5 mg in terms of teriparatide) was dissolved in 2480 g of the solution a, and its total amount was adjusted to 2500 mL with the solution a, to prepare Control Preparation 3.
Control Formulation 3 was subjected to filtration sterilization, and a sterile formulation was filled in a plastic syringe in an amount of 0.2 mL each, and a syringe filled with Control Formulation 3 was used as Control Formulation 3 Preparation. Here, Control Formulation 3 is a formulation having a volume of 0.2 mL, and having a teriparatide acetate concentration of 141 µg/mL in terms of teriparatide, and containing 28.2 µg of teriparatide acetate in a unit dose, in terms of teriparatide.

[Table 4]

Table 4

| Formulation | Additives | pH | Final Content |
|---|---|---|---|
| Control Formulation 3 | D-Mannitol: 136.5 g<br>Glacial acetic acid: 1230 mg<br>Sodium acetate hydrate: 498 mg | 4.1 | Teriparatide: 141 µg/mL<br>D-Mannitol: 45.5 mg/g<br>Glacial acetic acid: 0.41 mg/g<br>Sodium acetate hydrate: 0.166 mg/g |

(4) Preparation of Liquid Pharmaceutical Preparations Subjected to Test for Circular Dichroism (CD) Spectroscopy:

Formulations A to H were prepared in accordance with the following Table 5. Each of these formulations is nearly the identical formulation as Formulations A to H of "(2) Preparation of Liquid Pharmaceutical Preparations Subjected to Pharmacokinetic Tests in Human" mentioned above, from the viewpoint of their components.

A specific preparation method for each formulation is as follows. First, each additive listed in the column of "Additives" in the table was mixed together with a water for injection to prepare a solution **a** having a total volume of 3000 mL. Thereafter, teriparatide acetate (282 mg in terms of teriparatide) was dissolved in 1600 mL of the solution **a,** to prepare a drug solution **a.** Further, a diluted hydrochloric acid was added to the drug solution **a** to adjust its pH to that listed in the column of "pH" in the table, and a formulation of which entire volume was adjusted to 2000 mL with a water for injection was prepared.

Each formulation was subjected to filtration sterilization, and a sterile formulation was then filled in 2 mL ampules in an amount of 2 mL each, to produce ampules filled with each formulation (formulation ampule preparation), and subjected to a stability test relating to filled containers. In addition, each formulation was subjected to filtration sterilization, and a sterile formulation was then filled in a plastic syringe in an amount of 0.2 mL each, to produce a plastic syringe filled with each formulation (formulation syringe preparation), to be subjected to a stability test relating to filled containers.

The components of each formulation are as listed in the column of "Final Content."

[Table 5]

Table 5

| Formulation | Additives | pH | Final Content |
|---|---|---|---|
| Formulation A | Sodium chloride: 25.5 g<br>Purified white sugar: 75.0 g<br>L-Methionine: 300 mg | 4.6 | Teriparatide: 141 µg/mL<br>Sodium chloride: 8.5 mg/mL<br>Purified white sugar: 25 mg/mL<br>L-Methionine: 0.1 mg/mL<br>Hydrochloric acid: 0.1395 mM |
| Formulation B | Sodium chloride: 33.0 g<br>L-Methionine: 300 mg | 4.6 | Teriparatide: 141 µg/mL<br>Sodium chloride: 11 mg/mL<br>L-Methionine: 0.1 mg/mL<br>Hydrochloric acid: 0.134 mM |
| Formulation C | D-Mannitol: 180.0 g | 4.1 | Teriparatide: 141 µg/mL<br>D-Mannitol: 60 mg/mL<br>Hydrochloric acid: 0.208 mM |

(continued)

| Formulation | Additives | pH | Final Content |
|---|---|---|---|
| Formulation D | Purified white sugar: 312.0 g<br>L-Methionine: 300 mg | 4.1 | Teriparatide: 141 µg/mL<br>Purified white sugar: 104 mg/mL<br>L-Methionine: 0.1 mg/mL<br>Hydrochloric acid: 0.2135 mM |
| Formulation E | D-Mannitol: 90.0 g<br>Sodium chloride: 16.5 g<br>L-Methionine: 105 mg | 4.6 | Teriparatide: 141 µg/mL<br>D-Mannitol: 30 mg/mL<br>Sodium chloride: 5.5 mg/mL<br>L-Methionine: 35 µg/mL<br>Hydrochloric acid: 0.13 mM |
| Formulation F | D-Mannitol: 90.0 g<br>Sodium chloride: 16.5 g<br>L-Methionine: 300 mg | 4.6 | Teriparatide: 141 µg/mL<br>D-Mannitol: 30 mg/mL<br>Sodium chloride: 5.5 mg/mL<br>L-Methionine: 0.1 mg/mL<br>Hydrochloric acid: 0.1385 mM |
| Formulation G | D-Mannitol: 135.0 g<br>Purified white sugar: 78.0 g<br>L-Methionine: 105 mg | 4.1 | Teriparatide: 141 µg/mL<br>D-Mannitol: 45 mg/mL<br>Purified white sugar: 26 mg/mL<br>L-Methionine: 35 µg/mL<br>Hydrochloric acid: 0.205 mM |
| Formulation H | Sodium chloride: 16.5 g<br>Purified white sugar: 156.0 g<br>L-Methionine: 300 mg | 4.6 | Teriparatide: 141 µg/mL<br>Sodium chloride: 5.5 mg/mL<br>Purified white sugar: 52 mg/mL<br>L-Methionine: 0.1 mg/mL<br>Hydrochloric acid: 0.1375 mM |

(5) Preparation of Liquid Pharmaceutical Preparations Subjected to Stability Test:

Formulations A, B, E, F, and H were prepared in accordance with the following Table 6.

A specific preparation method for each formulation is as follows. First, each additive listed in the column of "Additives" in the table was mixed together with a water for injection to prepare a solution **a** having a total volume of 3000 mL. Thereafter, teriparatide acetate (282 mg in terms of teriparatide) was dissolved in 1600 mL of the solution **a,** to prepare a drug solution **a.** Subsequently, a diluted hydrochloric acid was added to the drug solution **a** to adjust its pH to that listed in the column of "pH" in the table, and a formulation of which entire volume was adjusted to 2000 mL with a water for injection was prepared.

Each formulation was subjected to filtration sterilization, and a sterile formulation was then filled in 2 mL ampules in an amount of 2 mL each, to produce ampules filled with each formulation (formulation ampule preparation), and subjected to a stability test. In addition, each formulation was subjected to filtration sterilization , and a sterile formulation was then filled in a plastic syringe in an amount of 0.2 mL each, to produce a plastic syringe filled with each formulation (formulation syringe preparation), to be used a stability test.

The components of each formulation are as listed in the column of "Final Content."

[Table 6]

Table 6

| Formulation | Additives | pH | Final Content |
|---|---|---|---|
| Formulation A | Sodium chloride: 25.5 g<br>Purified white sugar: 75.0 g<br>L-Methionine: 300 mg | 4.6 | Teriparatide: 141 μg/mL<br>Sodium chloride: 8.5 mg/mL<br>Purified white sugar: 25 mg/mL<br>L-Methionine: 0.1 mg/mL<br>Hydrochloric acid: 0.1395 mM |
| Formulation B | Sodium chloride: 33.0 g<br>L-Methionine: 300 mg | 4.6 | Teriparatide: 141 μg/mL<br>Sodium chloride: 11 mg/mL<br>L-Methionine: 0.1 mg/mL<br>Hydrochloric acid: 0.134 mg/mL |
| Formulation E | D-Mannitol: 90.0 g<br>Sodium chloride: 16.5 g<br>L-Methionine: 105 mg | 4.6 | Teriparatide: 141 μg/mL<br>D-Mannitol: 30 mg/mL<br>Sodium chloride: 5.5 mg/mL<br>L-Methionine: 35 μg/mL<br>Hydrochloric acid: 0.13 mM |
| Formulation F | D-Mannitol: 90.0 g<br>Sodium chloride: 16.5 g<br>L-Methionine: 300 mg | 4.6 | Teriparatide: 141 μg/mL<br>D-Mannitol: 30 mg/mL<br>Sodium chloride: 5.5 mg/mL<br>L-Methionine: 0.1 mg/mL<br>Hydrochloric acid: 0.1385 mM |
| Formulation H | Sodium chloride: 16.5 g<br>Purified white sugar: 156.0 g<br>L-Methionine: 300 mg | 4.6 | Teriparatide: 141 μg/mL<br>Sodium chloride: 5.5 mg/mL<br>Purified white sugar: 52 mg/mL<br>L-Methionine: 0.1 mg/mL<br>Hydrochloric acid: 0.1375 mM |

Example 2 (Pharmacokinetic Tests in Monkeys):

(1) Test Method:

Pharmacokinetic tests in monkeys were carried out using each of Formulations A to H prepared in "(1) Preparation of Liquid Pharmaceutical Preparations Subjected to Pharmacokinetic Tests in Monkeys" of Example 1 mentioned above, and Control Formulation 1 and Control Formulation 3 prepared in "(3) Preparation of Control Liquid Pharmaceutical Preparations" of Example 1 mentioned above.

Cynomolgus monkeys of ages from 4- to 6-years were subcutaneously administered with Formulations A to H, Control Formulation 1 and Control Preparation 3, and blood was collected from the veins of thighs at the time points of 5, 15, 30, 60, 120, and 180 minutes after the administration. PK tests were carried out in divided two tests (Tests 1 and 2). Each test had a crossover design, with a rest period appropriately set between each test period. Six animals were used per test. From blood obtained from these blood collections, plasma was collected by centrifugation, and a plasma teriparatide concentration was measured with an ELISA method (High Sensitivity Human PTH(1-34) ELISA kit, Immutopics Inc.). An area under the plasma concentration versus(-) time curve (AUC) was calculated on the basis of the plasma teriparatide concentration obtained by the measurement.

(2) Test Results:

The test results are shown in the following Tables 7 and 8.

[Table 7]

Table 7: Test 1 Pharmacokinetic Parameter

| Formulation | Formulation A | Formulation B | Formulation C | Formulation D | Control Formulation 1 | Control Formulation 3 |
|---|---|---|---|---|---|---|
| $AUC_{last}$ ng•min/mL | 243.7±52.8 | 316.8±137.7 | 161.0±55.7 | 135.6±31.6 | 273.7±57.5 | 182.4±36.5 |
| Number of Animals | 6 | 6 | 6 | 6 | 6 | 6 |

[Table 8]

Table 8: Test 2 Pharmacokinetic Parameter

| Formulation | Formulation E | Formulation F | Formulation G | Formulation H | Control Formulation 1 | Control Formulation 3 |
|---|---|---|---|---|---|---|
| $AUC_{last}$ ng•min/mL | 283.4 ± 72.6 | 243.3 ± 66.7 | 177.4 ± 48.3 | 285.9 ± 97.3 | 331.0 ± 44.4 | 185.8 ± 67.6 |
| Number of Animals | 6 | 6 | 6 | 6 | 6 | 6 |

As shown in Tables 7 and 8 mentioned above, the AUCs were shown to increase in the cases where Formulations A, B, E, F, and H were subcutaneously administered, as compared to AUC in the case where Control Formulation 3 was administered. In addition, the AUC in the case where Formulation B was administered was shown to increase as compared to Control Formulation 1. It was confirmed from the above results that in cynomolgus monkeys, Formulations A, B, E, F, and H showed even more favorable body pharmacokinetics, as compared to that of Control Formulation 3.

Example 3 (Pharmacokinetic Tests in Human):

(1) Test (1) Method:

Pharmacokinetic Tests in Human (1) were carried out using Control Formulation 2 and 3 Preparations prepared in "(3) Preparation of Control Liquid Pharmaceutical Preparations" of Example 1 mentioned above.

Specifically, pharmacokinetic tests was carried out in 12 cases of healthy postmenopausal women under unblinded tests, and Control Formulation 3 was subcutaneously administered in a unit dose to an abdominal part, a femoral part, or an upper arm part, out of which the pharmacokinetic parameter when administered to the abdominal part was compared to a pharmacokinetic parameter when Control Formulation 2 was subcutaneously administered to an upper arm part.

A plasma teriparatide acetate concentration was measured in blood samples collected before the administration of a formulation, and 5, 15, 30, and 45 minutes after the administration, and 1, 1.5, 2, 3, 4, and 6 hours after the administration. From the plasma teriparatide acetate concentration, pharmacokinetic parameters $AUC_{last}$, $AUC_{inf}$ and $C_{max}$ were each calculated for each subject in accordance with a method independent of any models, in accordance with the following formulas.

$AUC_{last}$ = Area under the plasma concentration versus(-) time curve in accordance with a linear trapezoidal rule until the last observation time

($AUC_{last}$ in Test (2) of Pharmacokinetic Tests in Human also being defined the same)

$AUC_{inf}$ = Area under the plasma concentration versus(-) time curve in accordance with a linear trapezoidal rule until infinitesimal time

($AUC_{inf}$ in Test (2) of Pharmacokinetic Tests in Human also being defined the same)

$C_{max}$ = Maximum plasma concentration

($C_{max}$ in Test (2) of Pharmacokinetic Tests in Human also being defined the same)

With respect to the calculated $AUC_{last}$, $AUC_{inf}$ and $C_{max}$, a ratio of Control Formulation 3 to Control Formulation 2 and a 95% confidence interval were calculated by the following method. With respect to $AUC_{last}$, $AUC_{inf}$ and $C_{max}$ each being logarithmically converted, analyses were made using variance analysis method according to mixed-effect models where subjects (in the order groups) were defined as random effects, the order group and the preparations (Control Formulation 2 and 3 Preparations) were defined as fixed effects. An estimated difference of the preparation and its 95% confidence interval were exponentially converted, and expressed in the form of a ratio between each formulation and its confidence interval.

In addition, as the evaluation items for safety, adverse events, clinical tests (blood tests, biochemical tests, urinary tests, immunological tests), vital signs (body temperatures at armpit, systolic and diastolic blood pressures, pulse rate), 12-inductive electrocardiogram, and body weight were provided, and the evaluation of safety by administration of Control Formulation 2 and 3 Preparations was carried out.

Twelve cases of subjects were randomly assigned to 4 groups of 3 cases each, and a test was carried out in accordance with the regimens as listed in the following Table 9 over 4 phases.

[Table 9]

Table 9

| Group | First Phase | Second Phase | Third Phase | Fourth Phase |
|-------|-------------|--------------|-------------|--------------|
| 1 | Control Formulation 2 | Control Formulation 3(*) | Control Formulation 3(**) | Control Formulation 3(***) |
| 2 | Control Formulation 3(**) | Control Formulation 2 | Control Formulation 3(***) | Control Formulation 3(*) |
| 3 | Control Formulation 3(*) | Control Formulation 3(***) | Control Formulation 2 | Control Formulation 3(**) |
| 4 | Control Formulation 3(***) | Control Formulation 3(**) | Control Formulation 3(*) | Control Formulation 2 |
| (*) abdominal part administration, (**) femoral part administration, (***) upper arm part administration. | | | | |

(2) Test (1) Results:

The test results are shown in the following Tables 10 and 11. $C_{max}$ when Control Formulation 3 was subcutaneously administered was about 1/2 of that when Control Formulation 2 was subcutaneously administered, and those of $AUC_{last}$ and $AUC_{inf}$ were about 1/4 (Table 11).

[Table 10]

Table 10: Pharmacokinetic Parameters

| Administration Formulation | $AUC_{last}$ (pg-hr/mL) | $AUC_{inf}$ (pg-hr/mL) | $C_{max}$ (pg/mL) |
|----------------------------|-------------------------|------------------------|--------------------|
| Control Formulation 2 | $969.3 \pm 201.4$ | $1079.1 \pm 190.8$ | $406.3 \pm 125.8$ |
| Control Formulation 3(***) | $258.9 \pm 124.3$ | $314.7 \pm 115.9$ | $186.8 \pm 69.0$ |
| (***) upper arm part administration | | | |

[Table 11]

Table 11: Ratio of Control Formulation 3 to Control Formulation 2 With Respect to Each Pharmacokinetic Parameter and Its 95% Confidence Interval

| Administration Formulation | $AUC_{last}$ Ratio (95% CI) | $AUC_{inf}$ Ratio (95% CI) | $C_{max}$ Ratio (95% CI) |
|----------------------------|------------------------------|-----------------------------|---------------------------|
| Control Formulation 3(***) | 0.28 (024,0.33) | 0.25 (0.21, 0.29) | 0.46 (0.37, 0.56) |
| (***) upper arm part administration | | | |

(3) Test (2) Method:

Pharmacokinetic Tests in Human (2) were carried out using each of Formulations A to H prepared in "(2) Preparation of Liquid Pharmaceutical Preparations Subjected to Pharmacokinetic Tests in Human" of Example 1 mentioned above, and Control Formulation 2 Preparation prepared in "(3) Preparation of Control Liquid Pharmaceutical Preparations" of Example 1 mentioned above.

Subjects were 24 healthy postmenopausal women. Under unblinded tests, the tests were carried out by comparing pharmacokinetic parameters obtained by subcutaneously administering Formulations A to H to an abdominal part in a unit dose with pharmacokinetic parameters obtained by subcutaneously administering Control Formulation 2 to an upper arm part.

The present tests were carried out in two cohorts: Groups I, II, III, and IV were a cohort 1, and Groups V, VI, VII, and VIII were a cohort 2. Twelve cases were randomly assigned to 4 groups of 3 cases each for each cohort. The subjects were administered with Formulations A to H and Control Formulation 2 in accordance with the regimens as listed in the following Table 12.

In Table 12, "-" means the fact that none of Formulations A to H and Control Formulation 2 were administered. The administration was carried out once in each phase, and the number of days of each phase was appropriately set in line with the purposes of the present test.

[Table 12]

Table 12

| Group | First Phase | Second Phase | Third Phase | Fourth Phase | Fifth Phase | Sixth Phase |
|---|---|---|---|---|---|---|
| I | Control Formulation 2 | Formulation A | Formulation B | Formulation C | Formulation D | - |
| II | Control Formulation 2 | Formulation C | Formulation A | Formulation D | Formulation B | - |
| III | Control Formulation 2 | Formulation E | Formulation F | Formulation G | Formulation H | - |
| IV | Control Formulation 2 | Formulation G | Formulation E | Formulation H | Formulation F | - |
| V | - | Formulation B | Formulation D | Formulation A | Formulation C | Control Formulation 2 |
| VI | - | Formulation D | Formulation C | Formulation B | Formulation A | Control Formulation 2 |
| VII | - | Formulation F | Formulation H | Formulation E | Formulation G | Control Formulation 2 |
| VIII | - | Formulation H | Formulation G | Formulation F | Formulation E | Control Formulation 2 |

A plasma teriparatide acetate concentration was measured using blood samples collected before the administration of a formulation, and 5, 15, 30, and 45 minutes after the administration, and 1, 1.5, 2, 3, 4, and 6 hours after the administration. From the plasma teriparatide acetate concentration, pharmacokinetic parameters $AUC_{last}$, $AUC_{inf}$ and $C_{max}$ were each calculated for each subject in accordance with a method independent of any models.

With respect to the calculated $AUC_{last}$, $AUC_{inf}$ and $C_{max}$, a ratio of Formulations A to H to Control Formulation 2 and a 95% confidence interval were calculated by the following method. First, the calculated $AUC_{last}$, $AUC_{inf}$ and $C_{max}$ were logarithmically converted, and thereafter analyses were made using variance analysis method according to mixed-effect models where subjects (in the order groups) were defined as random effects, and the order group and the formulations were defined as fixed effects. An estimated difference of the preparation and its 95% confidence interval were exponentially converted, and expressed in the form of a ratio between each formulation and its confidence interval.

Further, an absolute bioavailability rate (%) of plasma teriparatide was estimated using $AUC_{inf}$ (11.4 ng•min/mL) obtained by carrying out a different pharmacokinetic test in human using a teriparatide acetate preparation different from Formulations A to H (Non-Patent Publication 24; 2.7.1.2.2 Bioavailability), and $AUC_{inf}$ calculated from Formulations A to H and Control Formulation 3 mentioned above in accordance with the following formula.

[Math Formula 4]

Absolute Bioavailability Rate (%) of Component 1 =

$$\frac{\left\{\begin{array}{l}AUC_{inf}\ of\ Component\ 1\ Obtained \\ by\ Subcutaneous\ Administration\end{array}\right\} \times \left\{\begin{array}{l}Dosage\ of\ Component\ 1\ by \\ Intravenous\ Administration\end{array}\right\}}{\left\{\begin{array}{l}AUC_{inf}\ of\ Component\ 1\ Obtained \\ by\ Intravenous\ Administration\end{array}\right\} \times \left\{\begin{array}{l}Dosage\ of\ Component\ 1\ by \\ Subcutaneous\ Administration\end{array}\right\}} \times 100\ (\%)$$

Here, a different pharmacokinetic test mentioned above is a clinical pharmacological test which has a method of intravenously administering a teriparatide acetate preparation containing 14.1 μg in terms of teriparatide to 5 cases each of healthy men of ages in thirties and sixties continuously for 3 minutes, and the like.

In addition, the development of side effects was observed in the subjects administered with Formulations A to H (12 cases for each formulation) and the subjects administered with Control Formulation 2 (a total of 24 cases). The development rate (%) of the side effects was defined as a value calculated by dividing the number of individuals in which each side effect was developed by the number of individuals administered and multiplied by a factor of 100. Further, the serum calcium value elevation in the subjects administered with Formulations A to H and Control Formulation 2 was observed. The serum calcium value elevation was defined as a difference (mean) between a serum calcium value at 6 hours after the administration and a serum calcium value before the administration.

$T_{max}$ was calculated as a mean of the time at which a plasma teriparatide acetate concentration of each subject to be administered reached its maximum.

(4) Test (2) Results:

(4-1) Test:

The test results are shown in the following Tables 13 to 19. The formulations in which the upper limit of a 95% confidence interval of a ratio to Control Formulation 2 exceeded 0.5 were Formulations A, B, E, F and H in $AUC_{last}$, A, E, F and H in $AUC_{inf}$, and all of Formulations A to H in $C_{max}$. The order effects between Formulations A to H and Control Formulation 2 were not found (Table 14).

[Table 13]

Table 13: Pharmacokinetic Parameters (AUC, $C_{max}$)

| Administration Formulation | $AUC_{last}$ (pg-hr/mL) | $AUC_{inf}$ (pg-hr/mL) | $C_{max}$ (pg/mL) |
|---|---|---|---|
| Formulation A | 398.5 ± 81.5 | 430.2 ± 81.7 | 256.5 ± 117.7 |
| Formulation B | 400.1 ± 103.6 | 432.9 ± 101.0 | 265.4 ± 96.3 |
| Formulation C | 196.5 ± 52.9 | 230.6 ± 52.5 | 163.7 ± 74.9 |
| Formulation D | 231.0 ± 81.2 | 270.8 ± 72.3 | 158.0 ± 74.6 |
| Formulation E | 516.9 ± 113.0 | 548.7 ± 115.9 | 314.6 ± 105.5 |
| Formulation F | 525.7 ± 111.0 | 554.4 ± 106.7 | 338.0 ± 101.6 |
| Formulation G | 344.4 ± 129.5 | 376.8 ± 124.2 | 228.5 ± 105.0 |
| Formulation H | 503.5 ± 112.5 | 537.4 ± 112.5 | 322.5 ± 114.1 |
| Control Formulation 2 | 921.3 ± 148.2 | 1075.5 ± 209.3 | 335.9 ± 68.7 |

[Table 14]

Table 14: Ratio of Formulations A to H to Control Formulation 2 Relating to Each Pharmacokinetic Parameters (AUC, $C_{max}$) and Its 95% Confidence Interval

|  | $AUC_{last}$ Ratio (95% CI) | $AUC_{inf}$ Ratio (95% CI) | $C_{max}$ Ratio (95% CI) |
|---|---|---|---|
| Formulation A | 0.470 (0.408,0.542) | 0.442 (0.390,0.502) | 0.786 (0.662,0.933) |
| Formulation B | 0.463 (0.402, 0.534) | 0.439 (0.387,0.498) | 0.832 (0.701,0.988) |
| Formulation C | 0.233 (0.201,0.269) | 0.239 (0.210,0.272) | 0.506 (0.424,0.603) |
| Formulation D | 0.261 (0.227,0.301) | 0.274 (0.241,0.310) | 0.469 (0.395, 0.557) |
| Formulation E | 0.506 (0.439, 0.583) | 0.460 (0.406,0.522) | 0.827 (0.697, 0.982) |
| Formulation F | 0.516 (0.448,0.594) | 0.467 (0.412,0.529) | 0.903 (0.760,1.071) |
| Formulation G | 0.323 (0.280,0.372) | 0.307 (0.271,0.348) | 0.575 (0.484,0.682) |
| Formulation H | 0.492 (0.427,0.566) | 0.451 (0.397, 0.511) | 0.851 (0.717,1.010) |

(95% CI is 95% Confidence Interval.)

[Table 15]

Table 15: Pharmacokinetic Parameter (Absolute Bioavailability Rate)

| Administration Formulation | Absolute Bioavailability Rate (%) |
|---|---|
| Formulation A | 113.2 |
| Formulation B | 113.9 |
| Formulation C | 60.7 |
| Formulation D | 71.3 |
| Formulation E | 144.4 |
| Formulation F | 145.9 |
| Formulation G | 99.2 |
| Formulation H | 141.4 |
| Control Formulation 3 | 82.9 |

From the above results, it could be seen that Formulations A, B, E, F and H are preferred, from the viewpoint of pharmacokinetics.

[Table 16]

Table 16: Pharmacokinetic Parameter ($T_{max}$)

| Administration Formulation | $T_{max}$ (hr) |
|---|---|
| Formulation A | 0.5 |
| Formulation B | 0.5 |
| Formulation C | 0.25 |
| Formulation D | 0.25 |
| Formulation E | 0.5 |
| Formulation F | 0.625 |
| Formulation G | 0.25 |
| Formulation H | 0.5 |
| Control Formulation 2 | 0.75 |

[Table 17]

Table 17: Time course (hr) in a state of a plasma teriparatide acetate concentration of 100 pg/mL (or 250 pg/mL) or more in mean concentration-time profile

| Administration Formulation | Time Course in State of 100 pg/mL or More (hr) | Time Course in State of 250 pg/mL or More (hr) |
|---|---|---|
| Formulation A | 1.75 | 0 |
| Formulation B | 1.73 | 0.113 |
| Formulation C | 0.89 | 0 |
| Formulation D | 1.11 | 0 |
| Formulation E | 2.06 | 0.80 |
| Formulation F | 2.07 | 0.92 |
| Formulation G | 1.58 | 0 |
| Formulation H | 1.97 | 0.86 |
| Control Formulation 2 | 3.65 | 1.43 |

[Table 18]

Table 18: Development Rates (%) of Side Effects (Vomiting, Nausea, and Erythema at Injected Site)

| Administration Formulation | Development Rate of Nausea (%) | Development Rate of Vomiting (%) | Development Rate of Erythema at Injected Sites (%) |
|---|---|---|---|
| Formulation A | 0 | 0 | 41.7 |
| Formulation B | 16.7 | 8.3 | 33.3 |
| Formulation C | 8.3 | 0 | 50.0 |
| Formulation D | 0 | 0 | 58.3 |
| Formulation E | 16.7 | 0 | 41.7 |
| Formulation F | 8.3 | 0 | 25.0 |
| Formulation G | 16.7 | 0 | 58.3 |
| Formulation H | 8.3 | 0 | 50.0 |
| Control Formulation 2 | 41.7 | 25 | 25.0 |

In the subjects administered with Formulations A to H, adverse events such as headaches, abdominal flatulence, diarrhea, nausea, vomiting, and erythema at injected sites were found, and any other adverse events were not found at all. In addition, of these adverse events, headaches, nausea, vomiting and erythema at injected sites were found as side effects. The development rates of side effects of each of vomiting, nausea, and erythema at injected sites were as shown in the above Table 18.

[Table 19]

Table 19: Increased Level of Serum Calcium Concentration After 6 Hours of Administration (Based on Plasma Calcium Concentration Before Administration; mean)

| Administration Formulation | Increase in Serum Calcium Concentration (mg/dL) |
|---|---|
| Formulation A | 0.22 |
| Formulation B | 0.24 |
| Formulation C | 0.07 |

(continued)

| Administration Formulation | Increase in Serum Calcium Concentration (mg/dL) |
|---|---|
| Formulation D | 0.05 |
| Formulation E | 0.26 |
| Formulation F | 0.34 |
| Formulation G | 0.15 |
| Formulation H | 0.09 |
| Control Formulation 2 | 0.53 |

From the above results, it is said that the teriparatide acetate liquid preparations having smaller $T_{max}$, or a shorter time course in which the plasma teriparatide acetate concentration is a threshold value or more are generally excellent, from the viewpoint of the safety accompanying administration of a unit dose (in particular, side effects in digestive tracts).

Example 4 (Test for Circular Dichroism (CD) Spectroscopy):

(1) Test Method:

Using a circular dichroism dispersemeter (J-720; sold by JASCO CORPORATION), each of Formulation A to H Preparations prepared in "(4) Preparation of Liquid Pharmaceutical Preparations Subjected to Test for Circular Dichroism (CD) Spectroscopy" of Example 1 mentioned above, and Control Formulations 1 and 3 prepared in "(3) Preparation of Control Liquid Pharmaceutical Preparations" of Example 1 mentioned above was placed in a 1 mm cell, and the circular dichroism (CD) spectroscopy was carried out by 8 accumulations at 20°C. In addition, a placebo solution for each formulation was used as a blank solution.
The test was carried out in two runs, in which the circular dichroism spectroscopy was carried out for each of Control Formulation 1, Control Formulation 3, Formulation B, and Formulation D (a total of 4 formulations) as subjects to be measured in a measurement 1, and the circular dichroism spectroscopy was carried out for each of Formulations A to H (a total of 8 formulations) as subjects to be measured in a measurement 2.

(2) Test Results:

The test results are shown in the following Tables 20 to 22.

[Table 20]

Table 20: Measurement Results of Measurement 1

| | Control Formulation 3 | Formulation B | Formulation D |
|---|---|---|---|
| $\alpha$-Helix Content Ratio | 0.126 | 0.158 | 0.128 |
| Number of Amino Acid Residues That Form $\alpha$-Helix (number) | 4.284 | 5.372 | 4.352 |
| Average Residue Molar Ellipticity $[\theta]$ | -6144 | -7119 | -6221 |

[Table 21]

Table 21: Measurement Results (1) of Measurement 2

| | Formulation B | Formulation C | Formulation A | Formulation D |
|---|---|---|---|---|
| $\alpha$-Helix Content Ratio | 0.148 | 0.120 | 0.141 | 0.121 |
| Number of Amino Acid Residues That Form $\alpha$-Helix (number) | 5.032 | 4.08 | 4.794 | 4.114 |

(continued)

|  | Formulation B | Formulation C | Formulation A | Formulation D |
|---|---|---|---|---|
| Average Residue Molar Ellipticity [θ] | -6811 | -5968 | -6619 | -5998 |

[Table 22]

Table 22: Measurement Results (2) of Measurement 2

|  | Formulation E | Formulation F | Formulation G | Formulation H |
|---|---|---|---|---|
| α-Helix Content Ratio | 0.143 | 0.138 | 0.117 | 0.140 |
| Number of Amino Acid Residues That Form α-Helix (number) | 4.862 | 4.692 | 3.978 | 4.76 |
| Average Residue Molar Ellipticity [θ] | -6672 | -6512 | -5886 | -6570 |

Here, the term "average residue molar ellipticity [θ]" in the table refers to a numerical value in which a measurement value [m deg] at a wavelength of 222 nm was converted to a residue molar ellipticity ([deg.cm$^2$/d mol]), and the term "α-helix content ratio" refers to an α-helix content ratio estimated on the basis of the average residue molar ellipticity [θ] using the following mathematic formula.

[Math Formula 5]

$$\frac{\alpha\text{-Helix}}{\text{Content Ratio}} = \frac{-(\text{Average Residue Molar Ellipticity } [\theta] + 2340)}{30300}$$

(Non-Patent Publication 10)

The average residue molar ellipticities [θ] of Formulations A to H in the measurement results of the measurement 2 (measurement wavelength: 190 to 260 nm) are each shown in FIGs. 1A to 1H. Further, the average residue molar ellipticities [θ] of Formulations A to H in the measurement results of the measurement 2 (measurement wavelength: the portions of 210 to 230 nm) are shown in FIG. 1I.

In addition, in the measurement results of the measurement 2, the relationships between the average residue molar ellipticity $[\theta]_{222}$ and the $AUC_{last}$ Ratio (ratio of each formulation to Control Formulation 2 with respect to $AUC_{last}$) are shown in FIG. 2, and the relationships between the α-helix content ratio and the $AUC_{last}$ Ratio are shown in FIG. 3, respectively.

Each of Formulation A to H Preparations prepared in "Preparation of Liquid Pharmaceutical Preparations Subjected to Pharmacokinetic Tests in Human" mentioned above is nearly the identical formulation as Formulations A to H Preparations prepared in "Preparation of Liquid Pharmaceutical Preparations Subjected to Pharmacokinetic Tests in Monkeys" mentioned above. In view of the above, on the premises that the results for the pharmacokinetic tests in monkeys would hardly change even when the former Formulation A to H Preparations were exchanged with the latter Formulation A to H Preparations, the relationships between the α-helix content and the average residue molar ellipticity $[\theta]_{222}$ of teriparatide or a salt thereof contained in the liquid pharmaceutical composition in the present invention and the pharmacokinetic parameters of teriparatide or a salt thereof when the same composition was subcutaneously administered to monkeys were studied. The results are shown in FIGs. 4 and 5.

As is clear from the comparisons of these results and the results of the above Example 3, it could be seen that there is a clear correlation between the pharmacokinetics and the α-helix content ratio or the number of amino acid residues that form α-helix. Specifically, in the above Example 3, all of the formulations showing excellent pharmacokinetics (Formulations A, B, E, F, and H) showed larger values in the α-helix content ratios and the number of amino acid residues that form α-helix, as compared to the formulations besides them (Formulations C, D, G, and Control Formulation 3). By the utilization of the present invention, the inventors consider that liquid pharmaceutical compositions for subcutaneous administration in human containing teriparatide or a salt thereof having particularly remarkable pharmacokinetic properties can be acquired even more efficiently, economically advantageously, and safely, than conventional manners.

Example 5 (Stability Test):

(1) Test Method:

A stability test was carried out using Formulation A, B, E, F, and H Ampule Preparations prepared in "Liquid Pharmaceutical Preparations Subjected to Stability Test" mentioned above, and Formulation A, B, E, F, and H Syringe Preparations prepared in "Liquid Pharmaceutical Preparations Subjected to Stability Test" mentioned above, and the like.
Specifically, each of the formulation preparations was stored in a stability tester at 25°C / 60% RH. Thereafter, samples were taken on a third month, and subjected to high-performance liquid chromatography to measure stability.

(2) Test Results:

The test results are shown in the following Tables 23 and 24.
"Content Based on Initial Content" in the table shows a proportion (%) of the amount of teriparatide remaining at third month in a case where the amount of teriparatide before storage is defined as 100. "Total Amount of Analogs" in the table shows a proportion of a total amount of analogs which are present at third month in a case where the amount of teriparatide and the total amount of analogs which are present at third month is defined as 100.

[Table 23]

Table 23: Stability of Glass Ampule Preparations

| Formulation | Content Based on Initial Content | Total Amount of Analogs |
|---|---|---|
| Formulation A | 94.0 | 7.2 |
| Formulation B | 92.2 | 7.4 |
| Formulation E | 93.3 | 7.0 |
| Formulation F | 93.7 | 7.0 |
| Formulation H | 93.9 | 7.1 |

[Table 24]

Table 24: Stability of Plastic Syringe Preparations

| Formulation | Content Based on Initial Content | Total Amount of Analogs |
|---|---|---|
| Formulation A | 92.1 | 8.0 |
| Formulation B | 90.0 | 8.3 |
| Formulation E | 91.8 | 7.5 |
| Formulation F | 90.6 | 8.0 |
| Formulation H | 90.0 | 8.1 |

Example 6 (Simulation Test 1 on Pharmacokinetics):

A theoretical Component 1 containing preparation, the preparation having an absorption rate constant Ka of 0.48 (1/hr) obtained when the same preparation was administered subcutaneously in human in a unit dose (Preparation **a),** and a different theoretical Component 1 containing preparation, the preparation having an absorption rate constant Ka of 2 (1/hr) obtained when the same preparation was administered subcutaneously in human (Preparation **b)** were assumed, respectively. The influences of the changes in absorption rates on the plasma concentration transition of the Component 1 were confirmed by a simulation method utilizing a pharmacokinetic model that itself is known. For the pharmacokinetic model, a 1-compartment model with first-order absorption and elimination of analysis software Phenix WinNonlin 7.0 software (Certara: formerly Pharsight Corporation) was used. An outline of the 1-compartment model used in this example and Example 7 is schematically shown in FIG. 7. The clearances and the distribution volumes of each of Preparation **a** and Preparation **b** were assumed to be appropriately the same value, and the amounts of Component 1 contained in each of Preparation **a** and Preparation **b** were both 28.2 μg. The summary of the simulation results is shown in the following Table 25.

Here, in the 1-compartment model, the following formula (A) is applied.

[Math Formula 6]

$$C(T) = D*Ka/(V/F)/(Ka - Ke)*\{EXP(-Ke*T)) - EXP(-Ka*T)\}$$

$$\ldots \text{ formula (A)}$$

wherein T means time, Ka an absorption rate constant, Ke an elimination rate constant, V/F an apparent volume of distribution, C a concentration, D a dosage, respectively.

[Table 25]

Table 25

|  | Preparation **a** | Preparation **b** |
|---|---|---|
| AUC (hr*pg/ml) | 499.1 | 499.1 |
| $T_{max}$ (hr) | 0.538 | 0.315 |
| Time exceeding 100 pg/ml (hr) | about 2.4 | about 1.5 |
| Ka (l/hr) | 0.48 | 2 |

Example 7 (Simulation Test 2 on Pharmacokinetics):

(1) Test (1) Method:

On the bases of the results obtained by subjecting each of the preparations Nos. 1 to 12 listed in the following Table 26 to pharmacokinetic tests in human, each of V/F, Ka, and CL/F was calculated using a 1-compartment model in the same manner as in Example 6, and the relationship between Component 1 concentration in the preparation and Ka calculated was studied. Specifically, the Component 1 concentration (X) in the preparation and the calculated Ka (Y) were subjected to simple regression analysis, and a slope, an intercept, and a determination coefficient thereof were calculated. Here, Ka means an absorption rate constant, V/F a distribution volume, and CL/F a clearance, respectively, and a 1-compartment model is a model equivalent to the model in accordance with the formula (A) defined above.

[Table 26]

Table 26

| No. | Test | Name of Preparation | Component 1 Amount in Preparation (μg) | Component 1 Concentration in Preparation (μg/mL) | Measurement Value of $\alpha$-Helix Content of Component 1 in Preparation (%) |
|---|---|---|---|---|---|
| 1 | Ref. Ex. Clinical Test | Teriparatide 28.2 μg Preparation | 28.2 | 28.2 | Undetermined |
| 2 | Ref. Ex. Clinical Test | Teriparatide 56.5 μg Preparation | 56.5 | 56.5 | Undetermined |

(continued)

| No. | Test | Name of Preparation | Component 1 Amount in Preparation ($\mu$g) | Component 1 Concentration in Preparation ($\mu$g/mL) | Measurement Value of $\alpha$-Helix Content of Component 1 in Preparation (%) |
|---|---|---|---|---|---|
| 3 | Ex. 3 Test (1) | Formulation 2 Preparation | 56.5 | 56.5 | 13.0 or more |
| 4 | Ex. 3 Test (2) | Formulation A Preparation | 28.2 | 141.0 | 14.1 |
| 5 | Ex. 3 Test (2) | Formulation B Preparation | 28.2 | 141.0 | 14.8 15.8 |
| 6 | Ex. 3 Test (2) | Formulation E Preparation | 28.2 | 141.0 | 14.3 |
| 7 | Ex. 3 Test (2) | Formulation F Preparation | 28.2 | 141.0 | 13.8 |
| 8 | Ex. 3 Test (2) | Formulation H Preparation | 28.2 | 141.0 | 14.0 |
| 9 | Ex. 3 Test (2) | Control Formulation 2 | 56.5 | 56.5 | 13.0 or more |
| 10 | Test **a** | Formulation **a**-2 | 28.2 | 141.0 | 13.0 or more |
| 11 | Test **a** | Formulation **a**-3 | 28.2 | 141.0 | 13.0 or more |
| 12 | Test **a** | Formulation **a**-4 | 28.2 | 141.0 | 13.0 or more |
| (Here, Formulation **a**-2 to 4 are formulations prepared by filling a formulation of the preparation of any one of the above Formulation A to E Preparations in a different medical container.) | | | | | |

(2) Test (1) Results:

Ka of each preparation obtained by calculation using the 1-compartment model was as listed in the following Table 27. As a result of simple regression analysis using these Ka, a high correlation was found between a concentration (X) of Component 1 in the preparation and Ka (Y), as shown in the following mathematical formula.

[Math Formula 7]

$$Y \ (1/hr) = 0.0047X \ (\mu g/ml) + 0.3261$$

wherein $R^2 = 0.744$.

[Table 27]

Table 27

| No. | Ka (l/hr) |
|---|---|
| 1 | 0.57 |
| 2 | 0.51 |
| 3 | 0.70 |
| 4 | 1.01 |
| 5 | 1.22 |

(continued)

| No. | Ka (l/hr) |
|-----|-----------|
| 6 | 1.05 |
| 7 | 0.99 |
| 8 | 1.04 |
| 9 | 0.41 |
| 10 | 0.89 |
| 11 | 0.86 |
| 12 | 0.84 |

(3) Test (2) Method:

Further, Ka and Kel of each preparation obtained by calculation using the 1-compartment models (for Nos. 4 to 8 and 10 to 12 having a Component 1 concentration exceeding 100 μg/mL and a high bioavailability rate) were plugged into the following formula to calculate a theoretical $T_{max}$ for each preparation.

[Math Formula 8]

$$T_{max} = T_{max} = \ln (Ka / Kel) / (Ka - Kel)$$

provided that Ka ≠ Kel.

(4) Test (2) Results:

The calculated results were summarized in the following Table 28. As a result, a Ka width of each preparation was from 0.84 to 1.22. Here, $T_{max}$ of the Nos. 4 to 8 preparations obtained in accordance with methods independent of the model ($T_{max}$ of Formulations A, B, E, F, and H listed in Table 16 of Test Results (2) of Example 3) and theoretical $T_{max}$ of Nos. 4 to 8 preparations listed in the following table were not found to have a large dissociation, so that it is considered that each of the pharmacokinetic parameters (V/F, Ka, and CL/F) of each preparation obtained by calculation using the 1-compartment model are reasonable estimated values.

[Table 28]

Table 28

| No. | $T_{max}$ (hr) | Ka (l/hr) | Kel (l/hr) |
|-----|------|-----------|------------|
| 4 | 0.39 | 1.01 | 5.27 |
| 5 | 0.49 | 1.22 | 3.21 |
| 6 | 0.47 | 1.05 | 3.83 |
| 7 | 0.43 | 0.99 | 4.54 |
| 8 | 0.43 | 1.04 | 4.43 |
| 10 | 0.47 | 0.89 | 4.11 |
| 11 | 0.47 | 0.86 | 4.30 |
| 12 | 0.52 | 0.84 | 3.65 |

Further, when the maximum and minimum Ka (0.84 (1/hr) and 1.22 (l/hr)) of the above table were input into the above mathematical formula of simple regression analysis, the Component 1 concentration in the preparation was from 109 to 190 ($\mu$g/mL). $T_{max}$'s of Nos. 10 to 12 preparations obtained as a median in accordance with a method independent of the model are listed in the following Table 29.

[Table 29]

Table 29

| No. | $T_{max}$ (hr) |
|-----|----------------|
| 10 | 0.5 |
| 11 | 0.5 |
| 12 | 0.625 |

Reference Example (Reference Example Relating to Invention in Which $T_{max}$ of Component 1 Is Within Specified Range):

Clinical tests were carried out in 30 cases of healthy postmenopausal women under double blinded conditions, and pharmacokinetics, bone metabolism marker, and safety when teriparatide 28.2 $\mu$g or 56.5 $\mu$g was subcutaneously administered in a unit dose were compared with those of placebo.

The teriparatide 28.2 (or 56.5) $\mu$g preparation is an injection agent obtained by dissolving a teriparatide acetate containing freeze-dried preparation using 1 mL of Japanese Pharmacopoeia physiological saline upon use. Specifically, a teriparatide 28.2 $\mu$g preparation is a preparation having a volume of 1.0 mL, and containing 28.2 $\mu$g of teriparatide acetate in a unit dose, in terms of teriparatide. A teriparatide 56.5 $\mu$g preparation is a preparation having a volume of 1.0 mL, and contains 28.2 $\mu$g of teriparatide acetate in a unit dose, in terms of teriparatide.

A development rate (%) of side effects was defined as a value in which the number of individuals developing each of side effects was divided by the number of individuals administered and multiplied by a factor of 100. Further, a serum calcium value elevation was observed in the subjects administered with the teriparatide 28.2 (or 56.5) $\mu$g preparation. The serum calcium value elevation was defined as a difference (mean) between a serum calcium value at 6 hours after the administration and a serum calcium value before the administration.

$T_{max}$ was calculated as a mean of the time in which the plasma teriparatide acetate concentration of each individual to be administered reached its maximum.

The test results are shown in the following Tables 30 to 33.

[Table 30]

Table 30: Pharmacokinetic Parameter ($T_{max}$)

| Administration Formulation | $T_{max}$ (hr) |
|----------------------------|----------------|
| 28.2 $\mu$g Preparation | 0.9 |
| 56.5 $\mu$g Preparation | 0.875 |

[Table 31]

Table 31: Time course (hr) in a state that a plasma teriparatide acetate concentration is 100 pg/mL (or 250 pg/mL) or more, in mean concentration-time profile

| Administration Formulation | Time Course in a state of 100 pg/mL or more (hr) | Time Course in a state of 250 pg/mL or more (hr) |
|----------------------------|--------------------------------------------------|--------------------------------------------------|
| 28.2 $\mu$g Preparation | 2.24 | 0 |
| 56.5 $\mu$g Preparation | 3.69 | 1.43 |

[Table 32]

Table 32: Development Rates (%) of Side Effects (Vomiting, Nausea, and Erythema at Injected Site)

| Administration Formulation | Development Rate of Nausea (%) | Development Rate of Vomiting (%) | Development Rate of Erythema at Injected Sites (%) |
|---|---|---|---|
| 28.2 μg Preparation | 0 | 10 | 100 |
| 56.5 μg Preparation | 10 | 10 | 100 |
| Placebo | 0 | 0 | 0 |

[Table 33]

Table 33: Serum calcium concentration increased value at 6 hours after the administration (based on plasma calcium concentration before administration; mean)

| Administration Formulation | Serum Calcium Concentration Increase (mg/mL) |
|---|---|
| 28.2 μg Preparation | 0.31 |
| 56.5 μg Preparation | 0.47 |

INDUSTRIAL APPLICABILITY

The liquid pharmaceutical preparation of the present invention is excellent in the viewpoint of pharmacokinetics. The method of improving a pharmacokinetic parameter of the present invention is also an epoch-making main-drug controlling method. Therefore, the present invention is very useful in the medicament industries.

**Claims**

1. A liquid pharmaceutical preparation for subcutaneous administration in human comprising 28.2 μg of Component 1 in a unit dose in terms of teriparatide,
   the Component 1 being teriparatide or a salt thereof,
   wherein the Component 1 concentration is from 80 to 240 μg/mL.

2. The liquid pharmaceutical preparation for subcutaneous administration in human according to claim 1, wherein the Component 1 concentration is from 100 to 200 μg/mL.

3. The liquid pharmaceutical preparation for subcutaneous administration in human according to claim 1 or 2, wherein $T_{max}$ calculated by an analysis independent of pharmacokinetic models (NCA (Non Compartmental Analysis)) to the time of administration of a unit dose is from 0.5 to 0.7 (l/hr).

4. The liquid pharmaceutical preparation for subcutaneous administration in human according to any one of claims 1 to 3, wherein the time course in a state of a plasma concentration of the Component 1 of 100 pg/ml or more after administration of a unit dose is less than 2.1 (hr), and the time course in a state of a plasma concentration of the Component 1 of 250 pg/ml or more after administration of a unit dose is less than 1.0 (hr).

5. The liquid pharmaceutical preparation for subcutaneous administration in human according to any one of claims 1 to 4, for use in administration to postmenopausal women.

6. The liquid pharmaceutical preparation for subcutaneous administration in human according to any one of claims 1 to 5, wherein in the Component 1, the number of amino acid residues that form an α-helical structure is 4.5 or more and 5.5 or less.

7. The liquid pharmaceutical preparation according to claim 6, wherein the number of amino acid residues is the number of amino acid residues on the basis of the α-helix content ratio estimated using the following Estimation formula 1 from the numerical value a of the average residue molar ellipticity obtained by circular dichroism (CD) spectroscopy satisfying the following Measurement conditions 1 to 4:

Measurement condition 1: a measurement length of 222 nm;
Measurement condition 2: a sample concentration (Component 1 concentration) of from 0.1 to 0.3 mg/mL;
Measurement condition 3: a measurement temperature of 20°C; and
Measurement condition 4: a cell length of from 1 to 2 mm;

Estimation formula 1:

$$\alpha\text{-Helix Content Ratio} = \frac{-(\text{Numerical Value } \mathbf{a} + 2340)}{30300}.$$

8. The liquid pharmaceutical preparation for subcutaneous administration in human according to any one of claims 1 to 7, wherein the Component 1 is teriparatide acetate.

9. The liquid pharmaceutical preparation for subcutaneous administration in human according to any one of claims 1 to 8, wherein the liquid pharmaceutical preparation for subcutaneous administration in human is an aqueous pharmaceutical preparation for subcutaneous administration in human (excluding reconstructs of freeze-dried preparations).

10. The liquid pharmaceutical preparation for subcutaneous administration in human according to any one of claims 1 to 9, wherein the human liquid pharmaceutical preparation for subcutaneous administration is an aqueous pharmaceutical preparation for subcutaneous administration in human, and its solvent is a water for injection.

[FIG. 1A]

# FIG. 1A

[FIG. 1B]

# FIG. 1B

[FIG. 1C]

FIG. 1C

[FIG. 1D]

FIG. 1D

[FIG. 1E]

FIG. 1E

[FIG. 1F]

FIG. 1F

[FIG. 1G]

FIG. 1G

[FIG. 1H]

FIG. 1H

[FIG. 1I]

## FIG. 1I

[FIG. 2]

## FIG. 2

AUC$_{last}$ Ratio

Formulation E — Formulation F
Formulation B — Formulation H — Formulation A
Formulation G
Formulation D
Formulation C

Spectroscopy After Conversion to
Residue Molar Ellipticity (deg.cm$^2$/dmol)

[FIG. 3]

## FIG. 3

AUC$_{last}$ Ratio

Formulation F — Formulation E
Formulation H — Formulation B
Formulation A
Formulation G
Formulation D
Formulation C

α-Helix Content Ratio

[FIG. 4]

FIG. 4

AUC_last Ratio

Spectroscopy After Conversion to
Residue Molar Ellipticity (deg.cm²/dmol)

[FIG. 5]

FIG. 5

AUC_last Ratio

α-Helix Content Ratio

[FIG. 6]

FIG. 6

EP 3 685 850 A1

[FIG. 7]

FIG. 7

## INTERNATIONAL SEARCH REPORT

| International application No. |
|---|
| PCT/JP2018/034889 |

**A. CLASSIFICATION OF SUBJECT MATTER**
Int.Cl. A61K38/29(2006.01)i, A61K9/08(2006.01)i, A61P5/18(2006.01)i

According to International Patent Classification (IPC) or to both national classification and IPC

**B. FIELDS SEARCHED**

Minimum documentation searched (classification system followed by classification symbols)
Int.Cl. A61K38/29, A61K9/08, A61P5/18

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII)
CAplus/MEDLINE/EMBASE/BIOSIS /WPIDS (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2004-010511 A (ASAHI KASEI CORPORATION) 15 January 2004, claims 1, 3, paragraphs [0004], [0010], [0011] (Family: none) | 1, 2, 5, 8-10<br>3, 4, 6, 7 |
| Y | JP 5-306235 A (ASAHI KASEI INDUSTRY CO., LTD.) 19 November 1993, claim 1, paragraphs [0007], [0012] & US 5563122 A, claim 1, column 1, example 1 & WO 1993/011785 A1 & EP 619119 A1 | 1-10 |

☒ Further documents are listed in the continuation of Box C.      ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 14 December 2018 (14.12.2018) | 25 December 2018 (25.12.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/034889

C (Continuation).    DOCUMENTS CONSIDERED TO BE RELEVANT

| Category* | Citation of document, with indication, where appropriate, of the relevant passages | Relevant to claim No. |
|---|---|---|
| Y | WO 2011/030774 A1 (ASAHI KASEI PHARMA CORPORATION) 17 March 2011, claim 12, paragraphs [0023], [0037] & US 2012/0252729 A1, claim 12, paragraphs [0131], [0156] & EP 2476429 A1 | 1-10 |
| A | JP 2007-535466 A (THE GENERAL HOSPITAL CORPORATION) 06 December 2007, entire text & US 2006/0229240 A1 & WO 2004/067021 A1 & EP 1592434 A1 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI5306235 B
- JP 2004010511 A
- JP 2007186466 A
- JP 2001525372 A
- WO 200622301 A
- WO 2012169435 A
- JP 2015504087 A

- JP SHO6357527 B
- JP HEI296533 B
- JP 2004513069 A
- JP 2005213158 A
- WO 2011139838 A
- JP 2014507484 A

**Non-patent literature cited in the description**

- **SUNG et al.** *Journal of Biological Chemistry,* 1991, vol. 266 (5), 2831-2835
- **TAKEI et al.** *Peptide Chemistry 1979,* 1980, 187-192
- **MERRIFIELD.** *Advances In Enzymology,* 1969, vol. 32, 221-296
- **K. IKAWA et al.** *Jpn J Biomet,* 2015, vol. 36, S3-S18
- **MACH et al.** *Therapeutic Delivery,* 2011, vol. 2 (6), 727-736
- **KINNUNEN et al.** *Journal of Controlled Release,* 2014, vol. 182, 22-32
- Key Issues and Perspectives for Drug Metabolism and Pharmacokinetics in Drug Discovery and Development. *Sumitomo Chemical II,* 26-34
- **CHEN et al.** *Biochem. Biophys. Res. Commun.,* 1971, vol. 44 (6), 1285-1291
- **GREENFIELD.** *Nature Protocols,* 2006, vol. 1 (6), 2876-2890
- **LEE et al.** *Biopolymers,* 1989, vol. 28, 1115-1127
- **STRICKLAND et al.** *Biochemistry,* 1993, vol. 32, 6050-6057
- Proceedings of Annual Meeting of the Pharmaceutical Society of Japan, 118th Annual Meeting. 1998, vol. 4, 34
- **IZUTSU et al.** *Journal of Pharmaceutical Sciences,* 2006, vol. 95 (4), 781-789
- **H. HIRAMATSU.** Graduate School of Pharmaceutical Sciences and Faculty of Pharmaceutical Sciences. Tohoku University
- Secondary Structure Analysis of Proteins Using Infrared Absorption Spectroscopy. *The Society of Protein Science Archive,* 2009, vol. 2, e054

- **K. IZUTSU et al.** Tanpakushitu Iyakuhin no Hi-hakai-hyoka ni Muketa Suiyoeki to Toketsukanso-kotai-chu no Nijikozo Kento (Secondary Structure Studies on Protein Pharmaceutics in Aqueous Solutions and Freeze-Dried Solids Towards Nondestruction Evaluation). *Proceedings of 21st Near Infrared Forum Lectures,* 2005, 59
- **ARMSTRONG et al.** *Proc. Natl. Acad. Sci. USA,* 1993, vol. 90, 11337-11340
- **CHAKRABARTTY et al.** *Biochemistry,* 1993, vol. 32 (21), 5560-5565
- **WU et al.** *Proc. Natl. Acad. Sci. USA,* 1979, vol. 76 (8), 3656-3659
- **ALOJ et al.** *Archives of Biochemistry and Biophysics,* 1972, vol. 150 (2), 782-785
- **SALGIN et al.** *International Journal of Electrochemical Science,* 2012, vol. 7, 12404-12414
- **YAMAMOTO et al.** *Eur J Pharmacol.,* 2015, vol. 764, 457-462
- **MITSUHIRO MIYAZAWA.** Tokushu ni Atatte: Tanpakushitu no Rittaikozo Kaisekiho (Special Issue: Steric Structure Analysis Method of Proteins. *SAN-SHI-KONCHU BIOTEC,* 2012, vol. 81 (2), 105-106
- Standard Pharmacy Series 7: Science of Producing Preparations. Pharmaceutical Society of Japan, 10 February 2006, 12-13
- **N. KOSAKAYA et al.** Heikei-ki Nihonjin Josei niokeru Youtsui Kotsumitsudo no Gonenkan no Gensho ni Taisuru Kanren Inshi (Associating Factors for Loss in Lumbar Vertebrate Bone Density over a 5-Year Period in Menopausal Japanese Women). *Journal of Japan Society of Nutrition and Food Science,* 1999, vol. 52 (5), 307-313

- Maku Tokasei Pepuchido no Amino-san Hairetsu Kaihen niyoru pH Outousei no Hyoka (Evaluation of pH-Responsivity of Membrane-Permeable Peptides by Alterations of Amino Acid Sequences. **H. MIZUNO et al.** Outlines of 48th Academic Meeting Lectures. Nihon University, College of Industrial Technology, 05 December 2015, 543-544

- **TIM J et al.** *Protein Science,* 2007, vol. 16, 1193-1203
- **LEONID K.** 42. *Drug Metab. Dispos.,* 2014, 1890-1905